# FASCICULE DE BREVET EUROPEEN

(11) **EP 2 836 839 B1**
(45) Date de publication et mention de la délivrance du brevet: **04.10.2017**
(21) Numéro de dépôt: 12723219.7
(22) Date de dépôt: 12.04.2012
(51) Int. Cl.: G01N 33/68, G01N 33/76

(54) **PROCEDE ET TROUSSE DE DETECTION DU PIC PREOVULATOIRE DE LH**
VERFAHREN UND KIT ZUM NACHWEIS DES PRÄOVULATORISCHEN LH-ANSTIEGS
METHOD AND KIT FOR DETECTING THE LH PREOVULATORY SURGE

(43) Date de publication de la demande: 18.02.2015
(73) Titulaire: ReproPharm Vet, 37380 Nouzilly (FR)
(72) Inventeur: DECOURTYE, Jeremy, F-37360 Saint Antoine du Rocher (FR); DUPUY, Laurence, F-37110 Villedomer (FR); KARA, Elodie, F-37100 Tours (FR); MAUREL, Marie-Christine, F-37000 Tours (FR)
(74) Mandataire: Novagraaf Technologies
(86) Numéro de dépôt international: PCT/FR2012/050807
(87) Numéro de publication internationale: WO 2013/153291

(56) Documents cités:
- FR-A1- 2 662 804
- MORENO-ESCALLON B ET AL: "Luteinizing hormone in cervical mucus.", FERTILITY AND STERILITY APR 1982 LNKD- PUBMED:7067848, vol. 37, no. 4, avril 1982 (1982-04), pages 536-541, XP009160385, ISSN: 0015-0282
- HYUK KO ET AL: "Immunostick assay for medical diagnosis of rheumatoid arthritis", BIOTECHNOLOGY AND BIOPROCESS ENGINEERING, vol. 16, no. 6, 1 décembre 2011 (2011-12-01), pages 1248-1253, XP55030312, ISSN: 1226-8372, DOI: 10.1007/s12257-011-0106-7

## Description

### Domaine technique

La présente invention se rapporte à un procédé de détection du pic préovulatoire de LH dans un échantillon biologique provenant de mammifères et une trousse pour la mise en oeuvre du procédé de détection.

La présente invention trouve une application notamment dans le domaine médical et vétérinaire.

Dans la description ci-dessous, les références entre crochets (**[ ]**) renvoient à la liste des références présentée à la fin du texte.

### Etat de la technique

Le cycle de l'ovulation chez les mammifères fait intervenir un procédé hormonal complexe impliquant en particulier les hormones gonadotropes hypophysaires : l'hormone lutéinisante (LH) et l'hormone folliculo-stimulante (FSH).

En particulier, le cycle ovarien comporte deux phases : la phase folliculaire (croissance du follicule et maturation de l'ovocyte) aboutissant à l'ovulation, période propice à la fécondation, et la phase lutéale (mise en place du corps jaune). La FSH est responsable de la croissance et de la maturation du follicule préovulatoire et la LH induit la croissance folliculaire terminale de ce dernier et l'ovulation. En particulier, préalablement à l'ovulation, la LH et la FSH augmentent de manière significative dans le plasma jusqu'à un pic à la suite duquel l'ovulation intervient. Pour chaque espèce de mammifère, l'ovulation intervient à un moment très précis et constant après le pic de LH entre 12 et 52 heures après le pic de LH en fonction des espèces, 24 heures chez la vache, la brebis, la chèvre, la femme et 48 heures chez la truie, la chienne. Il est donc nécessaire et important de pouvoir disposer d'un test de détection du pic pré ovulatoire de LH qui soit robuste et utilisable dans les conditions d'élevage des animaux tout comme à la portée d'une utilisation humaine pour la femme. Ce test de détection du pic pré ovulatoire de LH permettra de prévoir le meilleur moment pour une insémination artificielle par exemple.

Il existe actuellement un test de détection de l'ovulation pour la chienne et la chatte, le test Witness® LH (marque déposée, commercialisé par la société Synbiotics corporation). Il est basé sur la détection de la LH et se réalise à partir du sérum uniquement. Toutefois, ce test n'est pas fiable à 100% et les résultats obtenus comprennent parfois un nombre significatif de faux positifs.

Il existe également, pour les êtres humains de sexe féminin, des tests d'ovulations basés, par exemple, sur une réaction d'agglutination DISCRETEST (marque déposée, commercialisé par la société CHEFARO ORGANON), ou des tests reposant sur une réaction immunoenzymatique, par exemple le test Clearblue, Test d'Ovulation Digital. Ces tests sont de type Lateral Flow et se présentent sous forme de bandelette, par exemple, le QuickTest™ (marque déposée), Babyprep Ovulation Test, ou sous forme de stylo ou de cassette, par exemple Polidis, Première Réponse, Conceive, Digitest. Tous se réalisent à partir de l'urine et permettent de détecter la présence (apparition de deux bandes colorées) ou l'absence de la LH (apparition d'une seule bande colorée). Toutefois, l'interprétation des résultats obtenus peut-être ambigüe, à savoir s'il y a présence ou absence d'une deuxième bande colorée, et présente un nombre significatif de faux négatifs ou faux positifs. Dans ce cas, ces procédés conduisent à une mauvaise interprétation et ne permettent pas de déterminer avec précision à quel moment va intervenir l'ovulation.

De plus, ces tests étant tous urinaires ils sont difficilement applicables pour des mammifères tels que les porcins, ovins, bovins, caprins ou nécessitent alors la présence d'une personne pour récolter l'urine du mammifère ce qui implique des coûts additionnels.

Deux autres types de tests existent pour la femme, l'un est basé sur la mesure du pH de la peau via la sueur par un microprocesseur maintenu en contact étroit avec la peau par un bracelet à scratch porté au poignet (OV-Watch® (marque déposée) et l'autre est basé sur l'observation, à l'aide d'un petit microscope, de salive séchée qui forme des motifs différents (observés au microscope) selon le taux d'estrogènes sécrétés au cours d'un cycle menstruel (donnaTEST® (marque déposée). Ces deux dispositifs sont onéreux et nécessitent un équipement particulier (bracelet ou petit microscope) inadapté pour les animaux et pour un usage simple chez la femme. En outre, le donnaTEST® (marque déposée) exige de faire l'examen sur de la salive parfaitement propre, prélevée à jeûn, ce qui est impossible avec les animaux. De plus, les paramètres mesurés (pH, taux d'oestrogènes) sont moins précis que le pic pré ovulatoire de LH pour une datation fiable de l'ovulation à 12 heures près.

En outre les procédés connus dans l'état de la technique pour les êtres humains ne sont pas directement applicables aux autres mammifères, en raison des spécificités différentes des anticorps utilisés et/ou du coût rédhibitoire qu'ils entraînent.

Il existe donc un réel besoin de trouver un procédé et un test palliant ces défauts, inconvénients et obstacles de l'art antérieur, en particulier un procédé permettant de détecter le pic de LH sur tout mammifère avec une spécificité et une sensibilité accrues. En outre il existe un réel besoin de trouver un procédé permettant une interprétation du résultat sans ambiguïté et fiable dans le temps. De plus, il existe également un réel besoin de trouver un procédé / test permettant de réduire les coûts des procédés de détection du pic de la LH et d'être adaptés aux conditions d'élevages. En particulier, il existe un réel besoin de trouver un procédé/test être utilisables à partir de milieux biologiques tels que le sang, le mucus vaginal, le mucus nasal, la salive l'urine ou le lait, et en n'exigeant pas des temps de révélation trop précis.

### Description de l'invention

La présente invention a pour but de surmonter les inconvénients de l'art antérieur en fournissant un procédé de détection du pic préovulatoire de LH dans un échantillon biologique provenant de mammifères et comprenant les étapes suivantes :
a. fixer un anticorps anti-LH sur une surface test ;
b. mettre en contact la surface test sur laquelle est fixé ledit anticorps anti-LH avec une solution tampon comprenant de 5 à 50% en volume de sérum de veau foetal;
c. mettre en contact ladite surface obtenue à l'étape (b) avec un échantillon biologique ;
d. après l'étape (c) rincer la surface test dans une solution de lavage ;
e. mettre en contact la surface test rincée à l'étape (d) avec une solution tampon de conjugué comprenant un anticorps anti-LH couplé à une enzyme et de 5 à 50% en volume de sérum de veau foetal ;
f. après l'étape (e) rincer la surface test dans une solution de lavage ; et
g. après l'étape (f) mettre en contact la surface test avec une solution comprenant un substrat de ladite enzyme, ledit substrat étant choisi dans le groupe comprenant le 3,3'3,3',5,5'-tétramethylbenzidine (TMB membrane), le tétrahydrochlorure de 4-Chloro-1-naphto / 3,3' - diaminobenzidine (CN/DAB), le 3-amino-9-éthylcarbazole (AEC), 3,3-Diméthoxybenzidine-o-dianisidine (ODN), le 5-bromo-4-chloro-3'-indolylohosphate pToluidine (BCIP), un mélange chlorure de nitro-bleu tétrazolium (NBT) et de 5-bromo-4-chloro-3'-indolylohosphate pToluidine (BCIP), le mélange Naphtol As-Mx phosphate et sel de 4-Chloro-2-méthylbenzènediazonium (Fast red TR salt), le mélange Naphtol As-Mx phosphate et le sel diazoté de chlorure de 4'-amino-2',5'-diéthoxybenzanilide zinc (Fast blue BB salt), le 5-Iodo-3-Indolyl-p-D-galactopyranoside (Purple-Gal), le 5-Bromo-6-chloro-3-indolyl-β-D-galactopyranoside (Red-Gal), le 6-chloro-3-indolyl-p-D-galactopyranoside (Rose-Gal), le 5-bromo-3-indolyl-p-D-galactopyranoside (Blue-Gal), le N-méthyndolyl-β-D-galactopyranoside (Green-Gal).
Le procédé de l'invention permet de détecter le pic préovulatoire de LH avec une spécificité et une sensibilité proches de 100% de celles d'un dosage quantitatif. Les inventeurs ont constaté découvert que le procédé de l'invention permet d'obtenir des résultats sans faux positif.

En outre, les inventeurs ont constaté que le procédé de l'invention permet la détection du pic de LH via une coloration de la surface test par le substrat de l'enzyme et que cette coloration est stable dans le temps.

Les inventeurs ont également constaté que l'intensité de coloration de la surface test par le substrat est proportionnelle à la concentration de la LH.

Le procédé de l'invention permet donc avantageusement de déterminer précisément la valeur seuil de la concentration de LH permettant, par exemple, de déterminer avec précision quand aura lieu l'ovulation.

Le procédé de l'invention permet également, avantageusement, de part la coloration de la surface test, une détection visuelle du pic de LH ne nécessitant pas de dispositif particulier pour la révélation et la lecture.

Selon l'invention, par pic préovulatoire de LH, on entend le pic d'hormone lutéinisante (LH) qui initie la rupture du follicule ovulatoire et provoque l'ovulation. Cela correspond par exemple une concentration de LH dans un liquide biologique supérieur à 0,5 ng/ml, par exemple chez la vache à une concentration de LH dans le plasma supérieure à 2ng/ml, par exemple chez la truie à une concentration de LH dans le plasma supérieur à 0,5 ng/ml .

Selon l'invention, par mammifère on entend un mammifère choisi dans le groupe comprenant l'ordre Monotremata, Didelphimorphia, Paucituberculata, Microbiotheria, Notoryctemorphia, Dasyuromorphia, Peramelemorphia, Diprotodontia, Tubulidentata, Sirenia, Afrosoricida, Macroscelidea, Hyracoidea, Proboscidea, Cingulata, par exemple le tatou, Pilosa, Scandentia, Dermoptera, Primates, Rodentia, Lagomorpha, Erinaceomorpha, Soricomorpha, Chiroptera, Pholidota, Carnivora, Perissodactyla, Artiodactyla et Cetacea.

Il peut s'agir par exemple d'un humain ou d'un animal. Il peut s'agir par exemple d'un animal d'élevage, d'un animal de compagnie, d'un animal en voie de disparition ou de tout autre animal dont la reproduction contrôlée présente un intérêt.

Par exemple, l'animal d'élevage peut être choisi dans le groupe comprenant les bovins, porcins, ovins, caprins, camelins, canins, équins, murins. Par exemple, l'animal de compagnie peut être choisi dans le groupe comprenant les canins et les félins.
Selon l'invention, le mammifère peut être, par exemple, un mammifère préalablement traité pour stimuler l'ovulation, par exemple avec un traitement de « superovulation » tel que décrit dans les références bibliographiques "Use of Norgestomet implant as an aid when superovulating low fertility dairy cattle", Ellington JE, Elefson EE, McCall RM. Theriogenology, 1987; 27, 227 [7] et « La production d'embryons chez les bovins : quelles voies de recherche pour augmenter l'efficacité des tratements de super ovulation », Saumande J. INRA Productions Animales, 1995 ; 8(4), 275-283 [6].

Selon l'invention, par « surface test » on entend une surface plane ou courbe ou rugueuse.

Selon l'invention, la surface test peut être une surface choisie parmi une surface en plastique, par exemple un polymère plastique, une surface en verre, une surface en polyméthylacrylate, une surface en polystyrène. De préférence, la surface test est une surface en plastique, par exemple un thermoplastique ou un thermodurcissable. Il s'agit de préférence d'une surface présentant des caractéristiques de couplage non spécifique très faible. Il peut s'agir par exemple de la surface MediSorp™ (marque de commerce), MaxiSorp™(marque de commerce), MultiSorp™(marque de commerce), MiniSorp™(marque de commerce) ou CovaLink™(marque de commerce).

Selon l'invention la surface test peut être par exemple une surface rectangulaire et/ou toute surface adaptée pour la mise en oeuvre du procédé. Il peut s'agir par exemple d'une spatule, d'un bâtonnet, d'une tige ou de tout autre support adapté à la mise en oeuvre de la présente invention. Il peut s'agir par exemple d'un bâtonnet ou « stick » à ailettes, par exemple de l'immunostick commercialisé par la société NUNC (Nunc Immuno™Stick (marque de commerce)).

Selon l'invention, par « échantillon biologique » on entend un échantillon liquide ou solide. Selon l'invention, l'échantillon peut être tout liquide biologique, par exemple, il peut s'agir d'un échantillon de sang, de plasma, de sérum, de mucus vaginal, de mucus nasal, de salive, d'urine et/ou de lait. De préférence l'échantillon biologique est un échantillon de sang ou un échantillon de mucus vaginal.

Selon l'invention, l'échantillon peut être un échantillon préalablement prélevé sur ledit mammifère.

Selon l'invention, l'étape a) de fixation d'un anticorps anti-LH sur une surface test peut être effectuée par tout procédé connue de l'homme du métier. Par exemple il peut s'agir d'un procédé décrit dans « Enzyme-linked immunosorbent assay (ELISA). Quantitative assay of immunoglobulin G », Engvall, E. et Perlman, P., Immunochemistry, 1971 Sep;8(9):871-4 [1]; «Techniques Immuno-enzymatiques » Thérèse Ternynck et Stratis Avrameas, éditions INSERM, 1987 [1]; « Immunobiologie » Charles A. Janeway, Paul Travers, Pierre L. Masson - 2003 - Medical ; Janeway's Immunobiology, Kenneth Murphy, Paul Travers, Mark Walport, 2011, éditions GS) [2].

Selon l'invention l'anticorps anti-LH peut être produit par tout procédé connu de l'homme du métier par exemple le procédé décrit dans la référence bibliographique, par exemple un procédé décrit dans « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ; 266 :2291-2294 [5]. Il peut s'agir par exemple d'un procédé comprenant l'immunisation d'un animal approprié à l'aide d'un antigène, par exemple une LH purifiée et/ou un anticorps anti-LH disponible dans le commerce.

Selon l'invention, l'anticorps anti-LH peut être un anticorps fait chez le lapin, l'ovin, le caprin, l'équin, le bovin, la souris, le rat. De préférence, l'anticorps anti-LH est un anticorps obtenu à partir d'un lapin.

Selon l'invention l'anticorps anti-LH fixé sur la surface test peut être un anticorps polyclonal ou monoclonal. Il peut s'agir d'un anticorps dirigé contre toute LH de mammifère, par exemple, il peut s'agir d'un anticorps anti-LH choisi parmi un anticorps anti-LH bovine, un anticorps anti-LH porcine, un anticorps anti-LH ovine, un anticorps anti-LH caprine, un anticorps anti-LH canine, un anticorps anti-LH féline, un anticorps anti-LH équine, un anticorps anti-LH cameline, un anticorps anti-LH humaine.

De préférence, l'anticorps anti-LH est un anticorps polyclonal anti-LH bovine, ou un anticorps polyclonal anti-LH porcine par exemple l'anticorps anti-LH bovine produit chez le lapin par la société Eurogentec (Belgique) selon le procédé «Standard anti-protein packages 28-day Speedy in Rabbit» (marque de commerce), l'anticorps anti-LH porcine peut être produit, par exemple par immunisation de lapins selon le procédé décrit par exemple dans la référence bibliographique « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ;266 :2291-2294 [5]. Il peut s'agir par exemple d'un procédé comprenant par exemple trois injections de 200 µg de LH porcine purifiée ont été réalisées toutes les deux semaines, suivi de dix rappels avec 100µg de LH purifiée effectués toutes les cinq semaines. Les saignées ont été réalisées six et neuf jours après chaque rappel. Ces anticorps de lapin ont été purifiés par exemple par chromatographie d'affinité sur gel de Protéine A sépharose selon le procédé décrit dans « Techniques Immuno-enzymatiques » Thérèse Ternynck et Stratis Avrameas, éditions INSERM, 1987 [1].

Les inventeurs ont découvert de manière inattendue que l'anticorps anti-LH bovine et l'anticorps anti-LH porcine permettent de détecter également la LH d'autres mammifères, par exemple la LH bovine, la LH porcine, la LH ovine, la LH caprine tout en conservant une spécificité et une sensibilité suffisantes au procédé de détection du pic préovulatoire de LH dans ces différentes espèces.

Selon l'invention, l'étape b) de mise en contact d'une surface test sur laquelle est fixée ledit anticorps anti-LH avec une solution tampon comprenant de 5 à 50% en volume de sérum de veau foetal peut être réalisée par immersion de ladite surface test dans ledit tampon, par aspersion de la surface test par ladite solution tampon.

Selon l'invention, l'étape b) de mise en contact d'une surface test sur laquelle est fixé ledit anticorps anti-LH avec une solution tampon comprenant de 5 à 50% en volume de sérum de veau foetal peut être réalisée pendant un temps supérieur à 30 minutes, par exemple de 30 minutes à 18 heures, par exemple de 1 à 18 heures.

Selon l'invention, la solution tampon comprenant de 5 à 50% en volume de sérum de veau foetal peut être choisie dans le groupe comprenant une solution tampon phosphate salin (PBS), une solution tampon tris salin (TBS), pH 7,4. De préférence la solution tampon comprenant de 5 à 50% en volume de sérum de veau foetal est une solution tampon K₂HPO₄/KH₂PO₄ 0,01M - NaCl 0,15M (PBS) pH 7,4.

Selon l'invention le sérum de veau foetal peut être du sérum de veau foetal disponible dans le commerce, par exemple il peut s'agir du sérum de veau foetal (SVF) commercialisé par la société Lonza sous la référence 14-801 F.

Selon l'invention, le pourcentage en volume de sérum de veau foetal dans la solution tampon à l'étape b) peut être compris de 5 à 50% en volume, de préférence de 20 à 50% en volume.

Selon l'invention, l'étape c) de mise en contact de ladite surface obtenue à l'étape (b) avec un échantillon biologique peut être réalisée par immersion de ladite surface test dans ledit échantillon, par aspersion de la surface test par ledit échantillon Selon l'invention, l'étape c) de mise en contact de ladite surface obtenue à l'étape (b) avec un échantillon biologique peut être réalisée pendant un temps supérieur à 5 minutes, par exemple de 5 à 60 minutes, par exemple pendant 15 minutes.

Selon l'invention, par échantillon biologique on entend un échantillon liquide ou solide. Selon l'invention, l'échantillon peut provenir de tout liquide biologique, par exemple, il peut s'agir d'un échantillon de sang, de plasma, de sérum, de mucus vaginal, de mucus nasal, de salive, d'urine et/ou de lait.

Selon l'invention, lorsque l'échantillon biologique est du sang, l'étape de mise en contact peut être réalisée par immersion de l'échantillon biologique et de la surface test dans une solution comprenant de l'héparine.

Selon l'invention, les étapes (d) et (f) de rinçage de ladite surface obtenue à l'étape (c) ou (e) avec une solution de lavage peuvent être réalisées par immersion de ladite surface test dans ladite solution de lavage, par aspersion de la surface test par ladite solution de lavage

Selon l'invention, les étapes (d) et (f) de rinçage de ladite surface obtenue à l'étape (c) ou (e) avec une solution de lavage peuvent être réalisées pendant un temps de 2 à 30 secondes, par exemple de 5 à 25 secondes, par exemple de 10 à 20 secondes.

Selon l'invention, la solution de lavage peut être toute solution de lavage connue de l'homme du métier et adaptée, par exemple il peut s'agir d'une solution choisie dans le groupe comprenant un tampon tris salin (TBS), un tampon phosphate salin (PBS), par exemple PBS pH 7,4 K₂HPO₄/KH₂ 0,01M - NaCl 0,15M, ou de l'eau.

Selon l'invention, la solution de lavage utilisée aux étapes (d) ou (f) peut être identique ou différente.

Selon l'invention, l'étape (e) de mise en contact d'une surface test avec une solution tampon de conjugué comprenant un anticorps anti-LH couplé à une enzyme et de 5 à 50% en volume de sérum de veau foetal peut être réalisée par immersion de ladite surface test dans ledit échantillon, par aspersion de la surface test par ledit échantillon.

Selon l'invention, l'étape e) de mise en contact de ladite surface peut être réalisée pendant un temps supérieur à 5 minutes, par exemple de 5 à 60 minutes, par exemple pendant 15 minutes.

Selon l'invention, la solution tampon de conjugué comprenant un anticorps anti-LH couplé à une enzyme et de 5 à 50% en volume de sérum de veau foetal peut être choisie parmi dans le groupe comprenant une solution tampon phosphate salin (PBS), une solution tampon tris salin (TBS), pH 7,4. De préférence la solution tampon de conjugué est un tampon K₂HPO₄/KH₂PO₄ 0,01M - NaCl 0,15M (PBS) pH 7,4.

Selon l'invention l'anticorps anti-LH couplé à une enzyme peut être un anticorps polyclonal ou monoclonal. Selon l'invention, l'anticorps anti-LH couplé à une enzyme peut être un anticorps de lapin, d'ovin, de caprin, d'équin, de bovin, de souris, de rat. Par exemple, l'anticorps anti-LH couplé à une enzyme peut être un anticorps anti-LH d'équin, par exemple un anticorps de cheval

Selon l'invention l'anticorps anti-LH couplé à une enzyme peut être un anticorps dirigé contre toute LH de mammifère, par exemple, il peut s'agir d'un anticorps anti-LH choisi parmi un anticorps anti-LH bovine, un anticorps anti-LH porcine, un anticorps anti-LH ovine, un anticorps anti-LH caprine, un anticorps anti-LH canine, un anticorps anti-LH féline, un anticorps anti-LH équine, un anticorps anti-LH cameline, un anticorps anti-LH humaine. Il peut s'agir par exemple d'un anticorps anti-LH canine, par exemple l'anticorps commercialisé par la société Millipore sous la référence catalogue AB944.

Selon l'invention, l'anticorps anti-LH est couplé à une enzyme choisie dans le groupe comprenant l'enzyme péroxydase, la béta-galactosidase, la glucose oxydase et la phosphatase alcaline. De préférence l'enzyme est la péroxydase, par exemple une péroxydase à hème ou une péroxydase sans hème. Il peut s'agir par exemple d'une peroxydase de raifort commercialisée par la société Sigma sous la référence catalogue P6782.

Selon l'invention, la solution tampon de conjugué peut être préparée préalablement ou concomitamment à la mise en oeuvre du procédé de l'invention.

Selon l'invention l'étape (g) de mise en contact de la surface test obtenue à l'étape (f) avec une solution comprenant un substrat de l'enzyme, peut être réalisée par immersion de ladite surface test dans ladite solution comprenant un substrat de l'enzyme, par aspersion de la surface test avec ladite solution comprenant un substrat de l'enzyme.

Selon l'invention (g) la mise en contact de ladite surface peut être réalisée pendant un temps supérieur à 5 minutes, par exemple de 5 à 15 minutes

Selon l'invention, la solution comprenant un substrat de l'enzyme peut être une solution tampon, par exemple une solution tampon tris salin (TBS), un tampon phosphate (K₂HPO₄/KH₂PO₄ 0,01M pH 7,4), une solution tampon phosphate salin (PBS), par exemple PBS pH 7,4 (K₂HPO₄/KH₂PO₄ 0,01M - NaCl 0,15M), de l'eau, un tampon citrate, par exemple citrate (NaCH₃CO₂) de 0,003 à 0,3M ajusté à pH5 avec de l'acide acétique (CH₃COOH). De préférence, la solution comprenant un substrat de l'enzyme est une solution tampon citrate (NaCH₃CO₂) à 0,03M ajusté à pH5.

Selon l'invention, le substrat peut être choisi dans le groupe comprenant le 3,3'3,3',5,5'-tétramethylbenzidine (appelé aussi TMB membrane), le tétrahydrochlorure de 4-Chloro-1-naphto / 3,3' - diaminobenzidine (appelé aussi CN/DAB), le 3-amino-9-éthylcarbazole (appelé aussi AEC), 3,3-Diméthoxybenzidine-o-dianisidine (appelé aussi ODN), le 5-bromo-4-chloro-3'-indolylohosphate pToluidine (appelé aussi BCIP), un mélange chlorure de nitro-bleu tétrazolium (appelé aussi NBT) et de 5-bromo-4-chloro-3'-indolylohosphate pToluidine (appelé aussi BCIP), le mélange Naphtol As-Mx phosphate et sel de 4-Chloro-2-méthylbenzènediazonium (appelé aussi Fast red TR salt), le mélange Naphtol As-Mx phosphate et le sel diazoté de chlorure de 4'-amino-2',5'-diethoxybenzanilide zinc (appelé aussi Fast blue BB salt), 5-lodo-3-indolyl-β-D-galactopyranoside (appelé aussi Purple-Gal), le 5-Bromo-6-chloro-3-indolyl-β-D-galactopyranoside (appelé aussi Red-Gal), le 6-chloro-3-Indolyl-β-D-galactopyranoside (appelé aussi Rose-Gal), le 5-bromo-3-Indolyl β-D-galactopyranoside (appelé aussi Blue-Gal), le N-méthylindolyl-β-D-galactopyranoside (appelé aussi Green-Gal).

De préférence, le substrat est le TMB membrane, de préférence le TMB membrane commercialisé par la société KPL sous la référence 50-77-18 ou par la société Sigma sous la référence T0565.

Selon l'invention le substrat peut être dilué dans ladite solution tampon avec un facteur de dilution de 1/2 à 1/20. De préférence, la dilution du substrat est de 1/2 à 1/8, égale à 1/2.

Avantageusement, la dilution du substrat, par exemple du TMB membrane, permet d'augmenter la visibilité de la détection du pic de LH en augmentant l'intensité de la coloration de la surface test.

Selon l'invention, le procédé de l'invention peut également comprendre après au moins une ou l'ensemble des étapes de rinçage, une étape de séchage. Il peut s'agir, par exemple, de l'incubation de la surface test dans une étuve à une température comprise de 25 à 37°C, par exemple à 37°C.

Selon l'invention, l'étape de séchage peut être réalisée pendant un temps de 0,5 à 5 heures, par exemple de 3 heures.

Selon l'invention, les étapes a) et b) du procédé peuvent être réalisées préalablement afin de disposer d'une surface test « prête à l'emploi ».

La présente invention a également pour objet une trousse pour la mise en oeuvre du procédé de l'invention, comprenant un support présentant une surface test sur laquelle des anticorps anti-LH sont fixés, un tampon PBS, une solution tampon de conjugués comprenant du sérum de veau foetal et un anticorps anti-LH couplé à une enzyme, et, une solution tampon de révélation comprenant un substrat de ladite enzyme, ledit substrat étant choisi dans le groupe comprenant le 3,3'3,3',5,5'-tétramethylbenzidine (TMB membrane), le tétrahydrochlorure de 4-Chloro-1-naphto / 3,3'- diaminobenzidine (CN/DAB), le 3-amino-9-éthylcarbazole (AEC), 3,3-Diméthoxybenzidine-o-dianisidine (ODN), le 5-bromo-4-chloro-3'-indolylohosphate pToluidine (BCIP), un mélange chlorure de nitro-bleu tétrazolium (NBT) et de 5-bromo-4-chloro-3'-indolylohosphate pToluidine (BCIP), le mélange Naphtol As-Mx phosphate et sel de 4-Chloro-2-méthylbenzènediazonium (Fast red TR salt), le mélange Naphtol As-Mx phosphate et le sel diazoté de chlorure de 4'- amino-2',5'-diéthoxybenzanilide zinc (Fast blue BB salt), le 5-lodo-3-Indolyl-p-D-galactopyranoside (Purple-Gal), le 5-Bromo-6-chloro-3-indolyl-β-D-galactopyranoside (Red-Gal), le 6-chloro-3-indolyl-p-D-galactopyranoside (Rose-Gal), le 5-bromo-3-indolyl-p-D-galactopyranoside (Blue-Gal), le N-méthyndolyl-β-D-galactopyranoside (Green-Gal). Selon l'invention la solution tampon de conjugués est telle que définie précédemment,

Selon l'invention, les anticorps anti-LH fixés sont tels que définis précédemment.

Selon l'invention, les anticorps anti-LH couplés à une enzyme sont tels que définis précédemment. Avantageusement, l'anticorps anti-LH couplé à une enzyme est un anticorps anti-LH couplé à la péroxydase

Selon l'invention, la solution de révélation est telle que définie précédemment.

Selon l'invention, le substrat de l'enzyme est tel que défini précédemment. Avantageusement, le substrat de l'enzyme est le TMB membrane.

Selon l'invention, le substrat de l'enzyme, lors de son oxydation permet avantageusement la coloration de la surface test et ainsi la détection du pic pré ovulatoire de LH.

Avantageusement, le procédé de l'invention et la trousse pour sa mise en oeuvre permettent une détection précise et semi-quantitative de la LH présente dans le liquide biologique. En particulier, selon l'invention, l'intensité de coloration de la surface test obtenue est proportionnelle à la concentration de la LH.

Avantageusement, selon l'invention, les substrats utilisés, par exemple le TMB membrane, permettent de colorer et de conserver la coloration sur la surface test après la mise en oeuvre du procédé.

Avantageusement, le procédé de l'invention permet une détection visuelle du pic de LH et ne nécessite pas de dispositif particulier, par exemple un dispositif optique.

Avantageusement, le procédé ainsi que la trousse de l'invention permettent de détecter très facilement, en ferme, le pic pré ovulatoire de LH et constituent un outil d'amélioration de la pratique de l'insémination artificielle chez les animaux. Par exemple, chez les animaux, elle est un outil de prédiction du moment de l'ovulation (intervenant 24 heures après le pic de LH) permettant ainsi de mieux planifier l'acte d'insémination artificielle, par exemple 12 heures après le pic de LH.

Le procédé et la trousse de l'invention peuvent ainsi être avantageusement utilisés en support/complément des traitements de super ovulation pour optimiser la fécondation et l'obtention d'un nombre important d'embryons de mammifère. En outre, cette optimisation permet avantageusement d'augmenter le nombre d'embryons de bonnes qualités et transférables.

D'autres avantages pourront encore apparaître à l'homme du métier à la lecture des exemples ci-dessous, illustrés par les figures annexées, données à titre illustratif.

### Brève description des figures

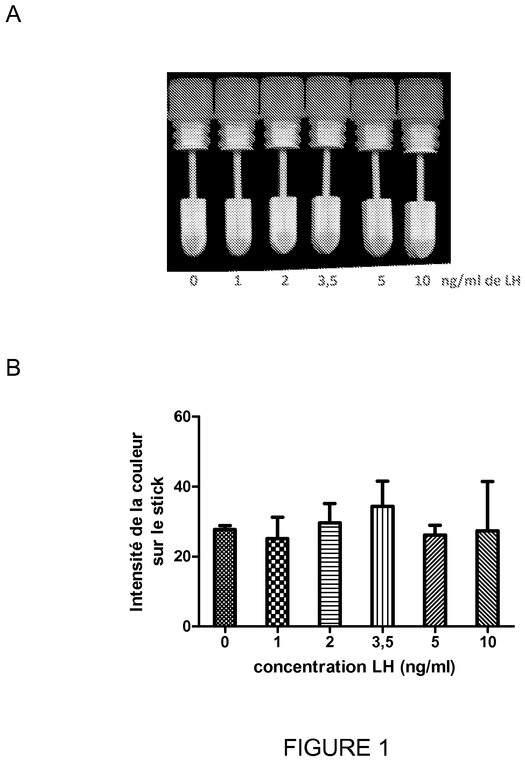

- La figure 1A représente une photographie de bâtonnets (« stick ») s après la mise en oeuvre du procédé de l'invention avec des plasmas bovins comprenant différentes concentrations de LH à savoir 0 ; 1 ; 2 ; 3,5 ; 5 et 10 ng/ml, les bâtonnets (« sticks ») s ayant été préalablement recouverts avec une solution tampon PBS.
- La figure 1B représente un diagramme en bâton ou histogramme de l'intensité de la couleur obtenue sur les bâtonnets (« sticks ») en unités de densité en fonction de la concentration en LH des plasmas.

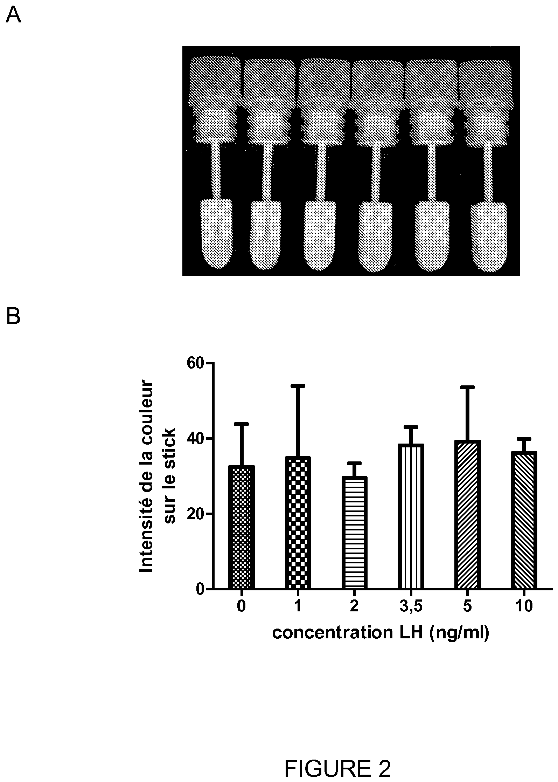

- La figure 2A représente une photographie de bâtonnets (« sticks ») après la mise en oeuvre du procédé de l'invention avec des plasmas bovins comprenant différentes concentrations de LH à savoir 0, 1 ; 2; 3,5; 5 et 10 ng/ml, les bâtonnets (« sticks ») ayant été préalablement recouverts avec une solution tampon PBS comprenant 50% de sérum de bélier hypophysectomisé.
- La figure 2B représente un diagramme en bâton de l'intensité de la couleur obtenue sur les bâtonnets (« sticks ») en unités de densité en fonction de la concentration en LH des plasmas.

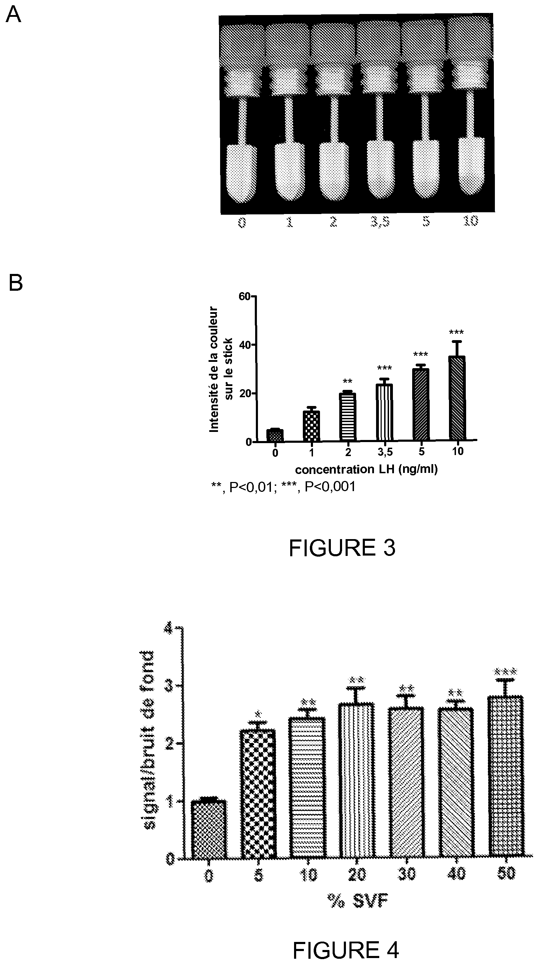

- La figure 3A représente une photographie de bâtonnets (« sticks ») après la mise en oeuvre du procédé de l'invention avec des plasmas bovins comprenant différentes concentrations de LH à savoir 0 ; 1 ; 2; 3,5; 5 et 10 ng/ml, les bâtonnets (« sticks ») ayant été préalablement recouverts avec une solution tampon PBS comprenant 20% de sérum de veau foetal.
- La figure 3B représente un diagramme en bâton de l'intensité obtenue sur les bâtonnets (« sticks ») en unités de densité en fonction de la concentration en LH des plasmas.
- La figure 4 représente un diagramme en bâton présentant le rapport entre l'intensité de la couleur des bâtonnets (« sticks ») obtenue après la mise en oeuvre du procédé de l'invention avec un plasma bovin ayant une concentration de LH de 7 ng/ml et celle obtenue avec un plasma bovin ayant une concentration de LH de 0 ng/ml. Les bâtonnets (« sticks ») ayant été préalablement recouverts avec une solution tampon PBS comprenant 0 ; 5 ; 10 ; 20 ; 30 ; 40 ; 50% en volume de sérum de veau foetal.

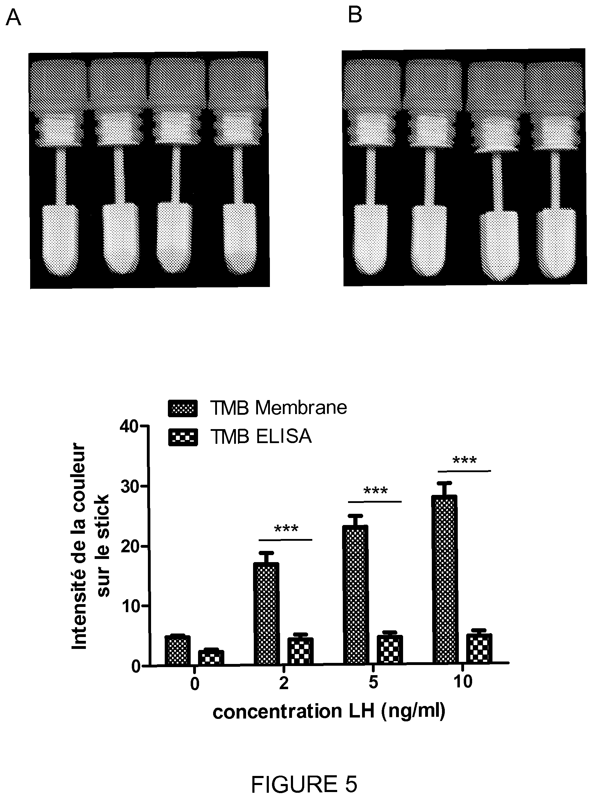

- La figure 5A représente une photographie de bâtonnets (« sticks ») après la mise en oeuvre du procédé de l'invention avec des plasmas bovins comprenant différentes concentrations de LH à savoir 0 ; 1 ; 2 ; 5 et 10 ng/ml après révélation avec le TMB membrane.
- La figure 5B représente une photographie de bâtonnets (« sticks ») après la mise en oeuvre du procédé de l'invention avec les mêmes plasmas bovins comprenant différentes concentrations de LH à savoir 0 ; 1 ; 2 ; 5 et 10 ng/ml après révélation avec le TMB ELISA.
- La figure 5C représente un diagramme en bâton de l'intensité de la couleur obtenue sur les bâtonnets (« sticks ») en unités de densité en fonction de la concentration en LH des plasmas. Les bâtons marqués avec un damier à grands carreaux représentent l'intensité de la couleur obtenue avec le TMB ELISA. Les bâtons marqués avec un damier à petits carreaux représentent l'intensité de la couleur obtenue avec le TMB Membrane.

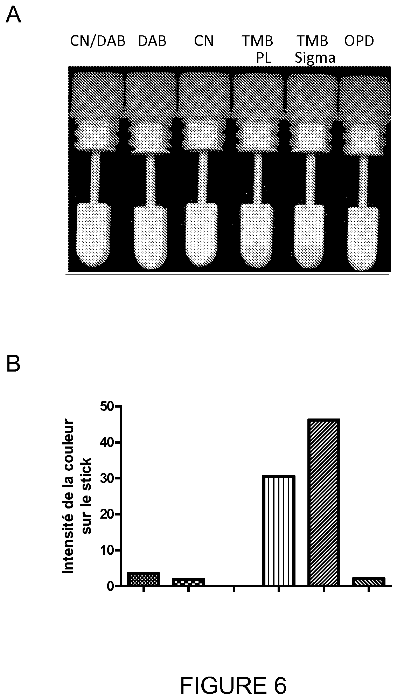

- La figure 6A représente une photographie de bâtonnets (« sticks ») après la mise en oeuvre du procédé de l'invention avec des échantillons de plasma bovin comprenant une concentration de LH de 19 ng/ml révélés avec différents substrats de l'enzyme péroxydase. Les substrats utilisés sont le tetrahydrochlorure de 3,3' - diaminobenzidine (DAB), le 4-chloro-1-naphtol (CN), le tétrahydrochlorure de 4-Chloro-1-naphto / 3,3' - diaminobenzidine (CN/DAB), l'ortho-phénylène-diamine (OPD), le TMB Membrane commercialisé par la société KPL (TMB KPL) ou par la société Sigma (TMB Sigma).
- La figure 6B représente un diagramme en bâton de l'intensité de la couleur obtenue sur les bâtonnets (« sticks ») en unités de densité en fonction des différents substrats de l'enzyme péroxydase : tétrahydrochlorure de 3,3' - diaminobenzidine (DAB), le 4-chloro-1-naphtol (CN), le tétrahydrochlorure de 4-Chloro-1-naphto / 3,3' - diaminobenzidine (CN/DAB), l'ortho-phénylène-diamine (OPD), le TMB Membrane commercialisé par la société KPL (TMB KPL) ou par la société Sigma (TMB Sigma).

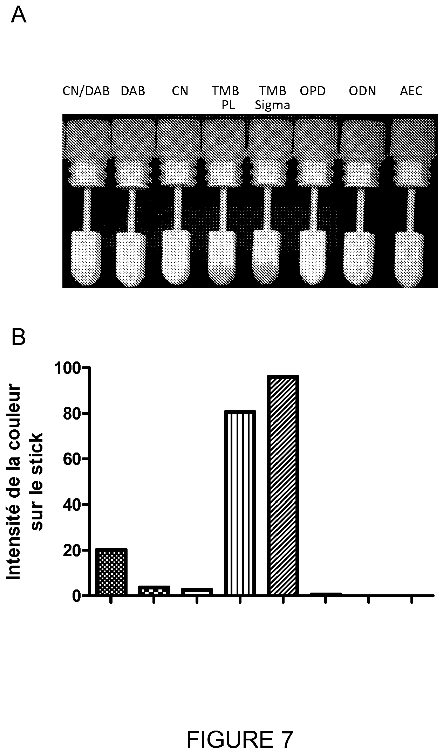

- La figure 7A représente une photographie de bâtonnets (« sticks ») après la mise en oeuvre du procédé de l'invention avec des échantillons de plasma bovin comprenant une concentration de LH de 19 ng/ml révélés avec différents substrats de l'enzyme. Les substrats utilisés sont le tétrahydrochlorure de 3,3' - diaminobenzidine (DAB), le 4-chloro-1-naphtol (CN), le tétrahydrochlorure de 4-Chloro-1-naphto / 3,3' - diaminobenzidine (CN/DAB), l'ortho-phénylène-diamine (OPD), le3,3-Dimethoxybenzidine,o-dianisidine (ODN), le3-amino-9-ethylcarbazole (AEC) le TMB Membrane commercialisé par la société KPL (TMB KPL) ou par la société Sigma (TMB Sigma).
- La figure 7B représente un diagramme en bâton de l'intensité de la couleur obtenue sur les bâtonnets (« sticks ») en unités de densité en fonction des différents substrats de l'enzyme : le tétrahydrochlorure de 3,3' - diaminobenzidine (DAB), le 4-chloro-1-naphtol (CN), le tétrahydrochlorure de 4-Chloro-1-naphto / 3,3' - diaminobenzidine (CN/DAB), l'ortho-phénylène-diamine (OPD), le 3,3-Diméthoxybenzidine,o-dianisidine (ODN), le 3-amino-9-éthylcarbazole (AEC) le TMB Membrane commercialisé par la société KPL (TMB KPL) ou par la société Sigma (TMB Sigma).

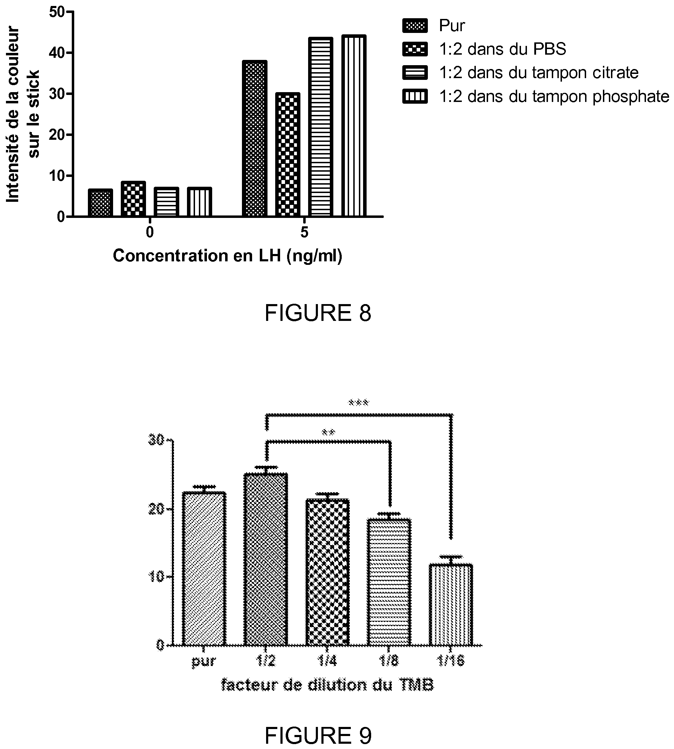

- La figure 8 représente un diagramme en bâton de l'intensité de la couleur obtenue sur les bâtonnets (« sticks ») en unités de densité en fonction de la concentration en LH des plasmas: 0 ou 5 ng/ml et en fonction de la dilution à 50% en volume du TMB membrane dans du PBS (K₂HPO₄/KH₂PO₄ 0,01M - NaCl 0,15M) pH 7,4 (bâtons avec les gros carrés); du tampon citrate (NaCH₃CO₂/CH₃COOH) 0,03M pH5 (bâtons hachurés horizontalement) ; du tampon phosphate 0,01M (K₂HPO₄/KH₂PO₄) pH 7,4 (bâtons hachurés verticalement), ou du TMB membrane sans dilution (pur) (bâtons avec les petits carrés).
- La figure 9 représente un diagramme en bâton de l'intensité de la couleur obtenue sur les bâtonnets (« sticks ») en unités de densité en fonction de la dilution au 1/2, 1/4, 1/8 et 1/16 du TMB membrane dans un tampon citrate (NaCH₃CO₂/CH₃COOH) 0,03M pH5, ou du TMB membrane sans dilution (pur).

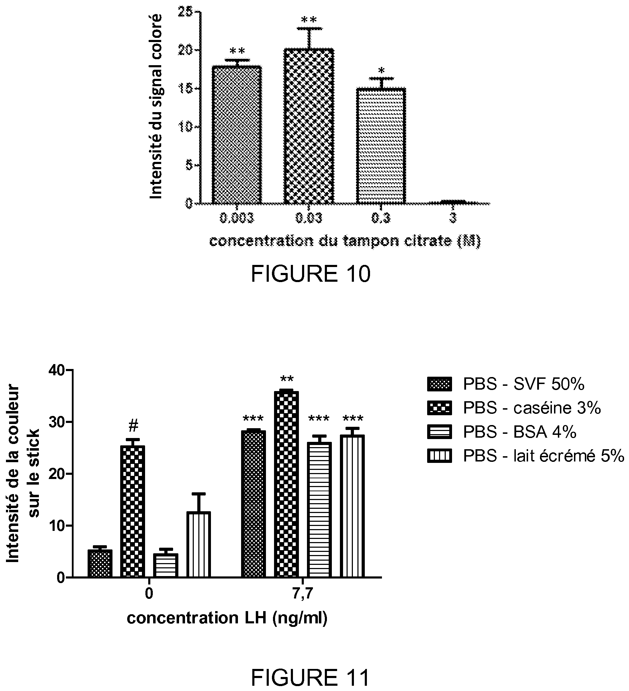

- La figure 10 représente un diagramme en bâton de l'intensité la couleur obtenue sur les bâtonnets (« sticks ») en unités de densité avec un plasma bovin de concentration en LH de 27 ng/ml, en fonction de la concentration du tampon citrate en molaire.
- La figure 11 représente un diagramme en bâton de l'intensité de la couleur obtenue sur les bâtonnets (« sticks ») en unités de densité en fonction de la concentration en LH : 0 ou 7,7ng/ml et en fonction du milieu protéique présent dans le tampon phosphate salin, à savoir du sérum de veau foetal à 50% en volume (bâtons avec des petits carrés), de la caséine à 3 % (30 g/L) (bâtons remplie avec des gros carrés), de l'albumine sérique bovine (BSA) à 4% (40g/L) (bâtons hachurés en horizontal), ou du lait écrémé à 5% (50 g/L) (bâtons hachurés horizontaux).

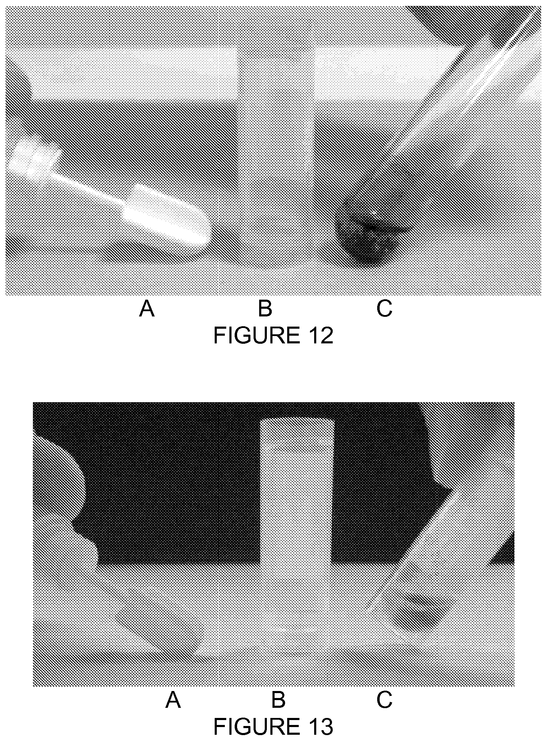

- La figure 12A représente une surface test (ailettes du bâtonnet (« stick »)) sensibilisée selon la mise en oeuvre du procédé avec de la glucose oxydase et immergée dans son substrat (PMS/NBT), la figure 12 B représente la solution de substrat dans laquelle la surface test a été immergée, la figure 12C représente une photographie d'un tube dans lequel la glucose oxydase en solution a été mise en présence de son substrat.
- La figure 13A est une photographie d'une surface test (ailettes du bâtonnet (« stick »)) selon la mise en oeuvre du procédé avec de la beta-galactosidase et immergée dans son substrat (X-gal), la figure 13 B représente la solution de substrat dans laquelle la surface test a été immergée, la figure 13C représente une photographie d'un tube dans lequel la beta-galactosidase en solution a été mise en présence de son substrat.

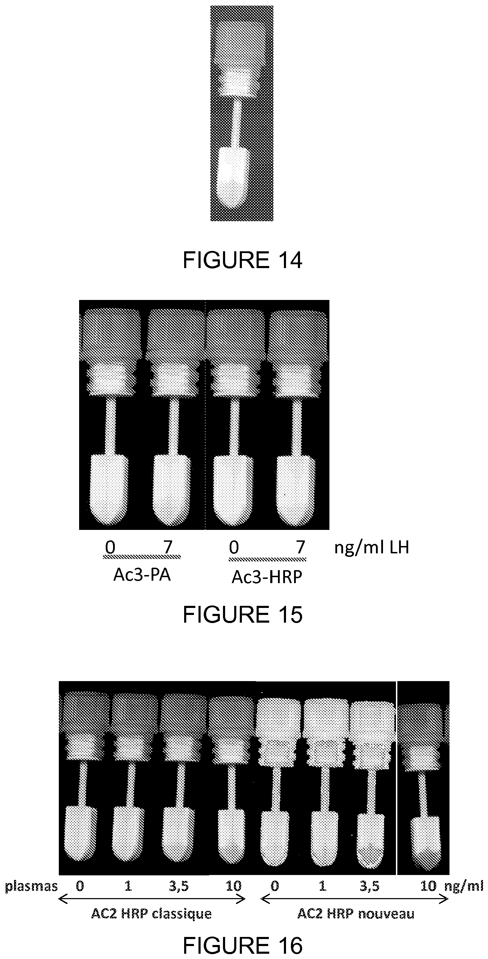

- La figure 14 est une photographie représentant un bâtonnet (« stick ») obtenu une surface test (ailettes du bâtonnet (« stick »)) selon la mise en oeuvre du procédé avec de la phosphatase alcaline.
- La figure 15 représente une photographie de bâtonnets (« sticks ») après la mise en oeuvre du procédé de l'invention avec des échantillons de plasmas bovins comprenant une concentration de LH de 0 ou 7 ng/ml (indiqué « 0 » et « 7 ») en fonction de l'enzyme couplée à un anticorps anti-IgG de cheval (AC3) à savoir la phosphatase alcaline (Ac3-PA) ou la péroxydase (Ac3-HRP). Dans ce cas, l'anticorps anti-LH de cheval (AC2) n'est pas couplé à une enzyme.
- La figure 16 représente une photographie de bâtonnets (« sticks ») après la mise en oeuvre du procédé de l'invention avec des échantillons de plasmas bovins comprenant une concentration de LH de 0 ; 1 ; 3,5 et 10 ng/ml en fonction de l'anticorps couplé à la péroxydase (désigné HRP) à savoir un anticorps anti-LH ovine fait chez le cheval indiqué AC2 HRP classique ou un anticorps anti-LH porcine fait chez le lapin indiqué AC2 HRP nouveau.

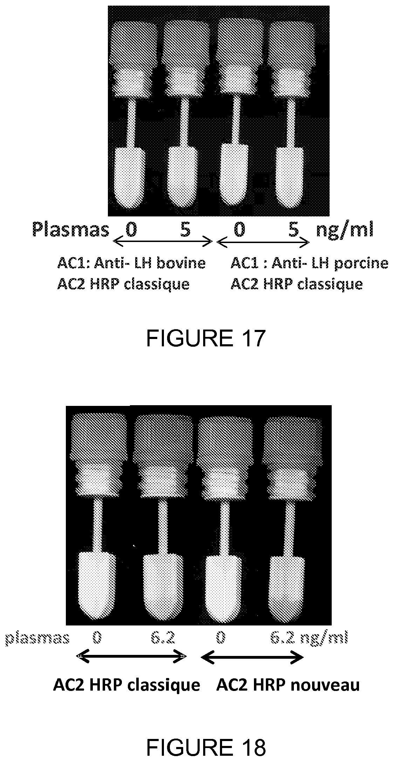

- La figure 17 représente une photographie de bâtonnets (« sticks ») après la mise en oeuvre du procédé de l'invention avec des échantillons de plasmas bovins comprenant une concentration de 0 ou 5 ng/ml de LH en fonction de l'anticorps fixé sur la surface test, à savoir un anticorps anti-LH bovine (AC1 : Anti-LH bovine) ou un anticorps anti-LH porcine (AC1 : Anti-LH porcine) faits chez le lapin.
- La figure 18 représente une photographie de bâtonnets (« sticks ») après la mise en oeuvre du procédé de l'invention avec des échantillons de plasmas bovins comprenant une concentration de 0 ou 6,2 ng/ml de LH en fonction de l'anticorps anti-LH couplé à l'enzyme utilisée, à savoir un anticorps anti-LH ovine fait chez le cheval (AC2 HRP classique) ou un anticorps polyclonal anti-LH porcine fait chez le lapin (AC2 HRP nouveau).

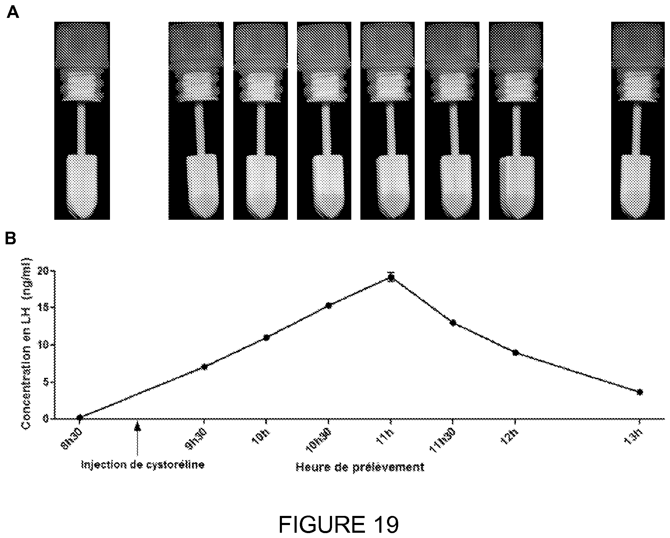

- La figure 19A représente des photographies de bâtonnets (« sticks ») obtenus après la mise en oeuvre du procédé en fonction de la concentration de la LH dans le sang d'une vache Holstein prélevé à différents temps.
- La figure 19B représente un diagramme de la valeur de la concentration plasmatique en LH en ng/ml en fonction de l'heure de prélèvement.

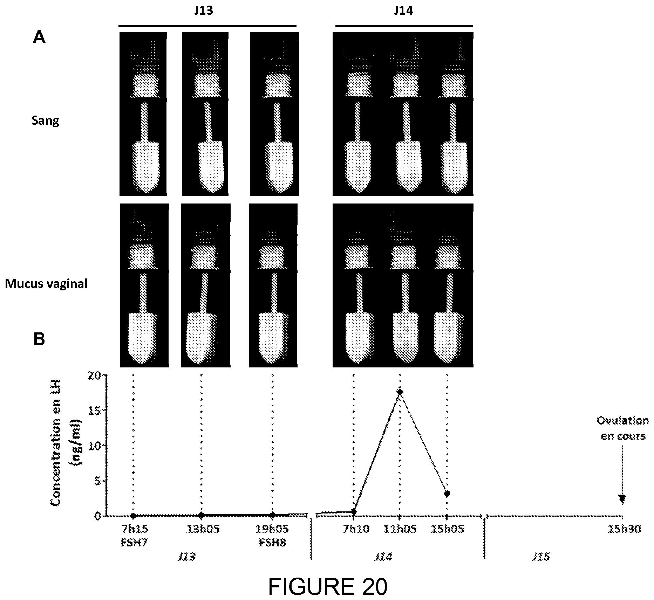

- La figure 20A représente des photographies de bâtonnets (« sticks ») obtenus selon le procédé de l'invention à partir du sang ou du mucus vaginal d'une vache ayant reçu un traitement de superovulation, au treizième (J13) et au quatorzième (J14) jour du cycle.
- La figure 20B représente la valeur de la concentration plasmatique en LH en ng/ml en fonction du temps en heures au treizième jour (J13) et quatorzième jour (J14) du cycle.

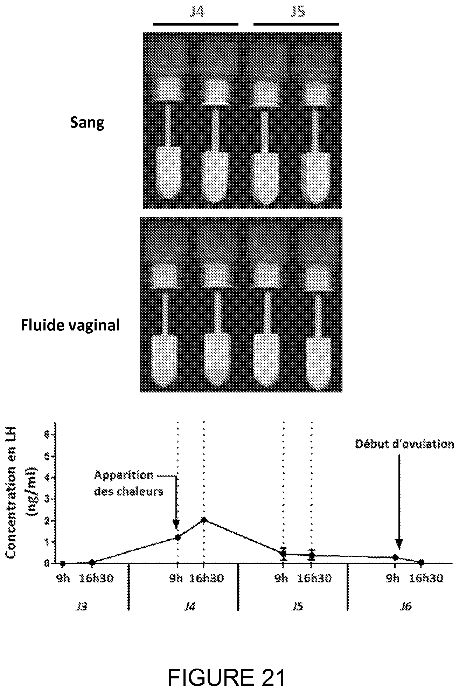

- La figure 21A représente des photographies de bâtonnets (« sticks ») obtenus selon le procédé de l'invention à partir du sang ou du mucus vaginal d'une truie (truie 1) au troisième jour (J3), quatrième jour (J4) et cinquième jour (J5) après sevrage. La figure 21B représente la concentration plasmatique en LH en ng/ml en fonction du temps en heures au troisième jour (J3), quatrième jour (J4) et cinquième jour (J5) après sevrage.

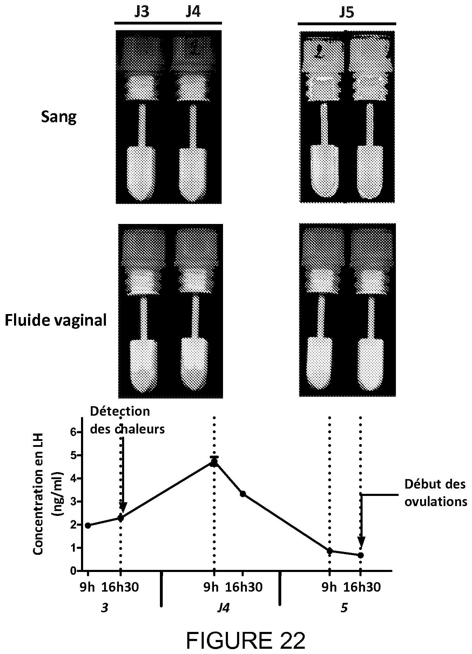

- La figure 22A représente des photographies de bâtonnets (« sticks ») obtenus selon le procédé de l'invention à partir du sang ou du mucus vaginal d'une truie (truie 2) au troisième jour (J3), quatrième jour (J4) et cinquième jour (J5) après sevrage.
- La figure 22B représente la concentration plasmatique en LH en ng/ml en fonction du temps en heures au troisième jour (J3), quatrième jour (J4) et cinquième jour (J5) après sevrage.

### EXEMPLES

### Exemple 1 : Procédé de détection d'un pic de LH.

Dans cet exemple, la surface test utilisée est une surface plastique d'un bâtonnet (« stick ») commercialisé par la société NUNC (ImmunoT"'Stick Nunc, Maxisorp).

Sur la surface test, un anticorps anti-LH a été fixé par recouvrement (coating) avec 250 µl d'anticorps lapin anti-LH bovine obtenu après purification sur colonne de Protéine A Sepharose à partir d'un sérum de lapin immunisé avec de la LH bovine purifiée dénommé ci-dessous AC1 préparé à 20 µg/ml dans du tampon NaHCO₃/Na₂CO₃ 0,1M pH 9,6 contenant 0,05% en volume de ProClin300 (marque déposée) commercialisé par la société Sigma-Aldrich, ref. 48912-U. La surface a ensuite été incubée pendant 1 heure à 37°C, puis 18 heures à 4°C.

La surface a ensuite été essorée par secousses pour éliminer le reste du liquide d'AC1.

La surface test sur laquelle est fixé l'anticorps a été mise en contact, via le recouvrement (« surcoating ») de la surface avec 900µl de PBS additionné de Sérum de Veau Foetal (SVF) 50% en volume (Lonza, référence 14-801F) pendant 1 heure à 37°C. Ladite surface obtenue a été ensuite essorée par secousses et séchée à la verticale dans une étuve à 37°C pendant 3 heures.

La surface obtenue a été mise en contact avec un échantillon de 250µl de sang par immersion dans un tube hépariné (cryotube en polypropylène NUNC, ref. 368632). Le tube hépariné a été préalablement traité par 12 µl d'une solution d'héparine à 500UI/ml préparée à partir de l'Héparine Choay commercialisée par Sanofi Aventis et diluée dans du PBS filtré sur un filtre de 0,2µm de porosité (Millipore, ref. GSWP04700), puis séché pendant 3 heures dans une étuve à 37°C.

La surface test a été ensuite rincée dans 30 mL d'une solution de lavage, à savoir une solution tampon phosphate salin PBS pH 7,4 K₂HPO₄/KH₂PO₄ 0,01M - NaCl 0,15M comprise dans un tube de 40 ml La surface test rincée obtenue a été mise en contact dans un cryotube (commercialisé par la société NUNC, ref. 368632) avec 300µl d'une solution tampon de conjugué comprenant un anticorps de cheval anti-LH ovine couplé à la péroxydase de raifort (HRP) préparé selon le procédé décrit dans « Techniques Immuno-enzymatiques » (Thérèse Ternynck et Stratis Avrameas, éditions INSERM, 1987) dénommé ci-dessous AC2 HRP à 10µg/ml dans du PBS SVF 50% en volume contenant 0,05% en volume de ProClin300 (marque déposée) préalablement préparé. Il s'agissait en particulier de l'anticorps l'AC2 HRP fait chez le cheval décrit dans le brevet français FR N° FR90 06863, N° de brevet FR 2 662 804

La surface test a été ensuite rincée dans 30 mL d'une solution de lavage, à savoir une solution tampon phosphate salin PBS pH 7,4 K₂HPO₄/KH₂PO₄ 0,01M - NaCl 0,15M comprise dans un tube de 40 ml

La surface test rincée obtenue a été mise en contact pendant 15 minutes dans un cryotube commercialisé par la société NUNC, ref. 368632 avec une solution comprenant un substrat de péroxydase de raifort (HRP) à savoir 300µL de TMB Membrane (commercialisé par la société KPL, ref. 50-77-18) dilué à 50% dans du tampon citrate (NaCH₃CO₂) 0,03M ajusté à pH5 avec de l'acide acétique (CH₃COOH).

La surface obtenue a été ensuite retirée du tube et l'observation visuelle de la coloration bleue de la surface test a été effectuée et a montré la présence de LH dans l'échantillon testé.

Tel que démontré dans cet exemple, le procédé de l'invention permet avantageusement de détecter la présence de LH dans un échantillon biologique.

### Exemple 2 : essais comparatifs avec différents constituants

Dans cet exemple, les produits/procédés utilisés sont ceux de l'exemple 1 à l'exception des éléments ci-dessous.

### 1. Test de tampons de recouvrement (« surcoatinq ») et de préparation de l'anticorps couplé à l'enzyme (AC2-HRP)

Trois tampons ont été testés :
- du PBS pH 7,4 (K₂HPO₄/KH₂PO₄ 0,01M - NaCl 0,15M) (VWR, ref. 26930.293, 26936.293, 27810.295)
- du PBS comprenant 20% en volume de SVF (sérum de veau foetal) (Lonza, référence 14-801F)
- du PBS comprenant 50% en volume de sérum de bélier hypophysectomisé (milieu INC - demande de brevet N° FR90 06863, INRA/CNRS, N° de brevet FR 2 662 804).

Ces trois tampons ont servi à réaliser la mise en contact de la surface test pour son recouvrement (« surcoating ») et à diluer l'anticorps anti-LH ovine couplé à la péroxydase, l'AC2-HRP à savoir un anticorps anti-LH purifié sur colonne de Protéine G Sépharose à partir d'un sérum de cheval immunisé contre la LH ovine et couplé avec de la péroxydase (Sigma, référence P6782). Il s'agissait en particulier de l'anticorps l'AC2 HRP fait chez le cheval décrit dans le brevet français FR N° FR90 06863, N° de brevet FR 2 662 804.

Les conditions de fabrication du bâtonnet (« stick ») sont celles décrites ci-dessus.

Chacune des trois conditions a été évaluée sur 6 plasmas prélevés de façon sériée sur une même vache en période de pic préovulatoire de LH. Ces plasmas ont été préalablement dosés quantitativement par méthode ELISA, avec le kit LH DETECT (marque déposée) (ReproPharm SA, France). Leurs concentrations en LH étaient respectivement de 1 - 2 - 3,5 - 5 et 10 ng/ml. Ces expériences ont été répétées entre trois et cinq fois. A chaque expérience, la lecture visuelle des résultats a été validée par une quantification de la couleur obtenue sur les bâtonnets (« sticks »). Pour cela, les bâtonnets (« sticks ») sont scannés à l'aide d'un Scanner EPSON (Perfection 1200 PHOTO) puis l'intensité de la couleur obtenue sur chacun d'eux est quantifiée par densitométrie avec le logiciel « Scion Image » (Scion Incorporation). Cette quantification est exprimée en unités de densité. La comparaison statistique des résultats a été faite par analyse de variance à un facteur suivi d'un test de Bonferroni ou par le test de Kruskall-Wallis en utilisant le logiciel GraphPad Prism version 5.

Les résultats obtenus avec du PBS sont illustrés sur la figure 1 et dans le tableau 1 ci-dessous. La figure 1A représente une photographie de bâtonnets (« sticks ») après la mise en oeuvre du procédé de détection avec des plasmas de vache comprenant différentes concentrations de LH à savoir 0 ; 1 ; 2 ; 3,5 ; 5 et 10 ng/ml, les bâtonnets (« sticks ») ayant été préalablement recouverts avec une solution tampon PBS. La figure 1 B représente un diagramme en bâton de l'intensité de la couleur obtenue sur les bâtonnets (« sticks ») en unités de densité en fonction de la concentration de LH. Tel que représenté sur la figure 1A, une couleur bleue intense est observée sur l'ensemble des bâtonnets (« sticks ») quel que soit la concentration en LH du plasma. Un fort signal coloré a été également observé avec le plasma négatif (0ng/ml de LH) ce qui traduit par un fort signal non-spécifique très défavorable dans la mise au point du test.

**Tableau 1 : intensité de la couleur en fonction de la concentration de LH avec le tampon PBS**

| Concentration de LH (ng/ml) | Intensité de la couleur (unités) | |
|---|---|---|
| | Moyenne | Ecart-type |
| 0 | 27,7 | 1,09 |
| 1 | 25,14 | 6,04 |
| 2 | 29,59 | 5,5 |
| 3,5 | 34,28 | 7,18 |
| 5 | 26,11 | 2,75 |
| 10 | 27,21 | 14,16 |

Tel que démontré dans cet exemple, la couleur apparue sur le bâtonnet (« stick ») est homogène mais indépendante de la concentration de LH (figure 1A). Le plasma sans LH (0 ng/ml) donne une forte couleur et témoigne d'un fort signal non-spécifique. Il n'y a aucune différence significative entre les bâtonnets (« sticks ») lorsque le tampon PBS est utilisé pour recouvrir la surface test et pour diluer l'anticorps anti-LH couplé à l'enzyme.

Les résultats obtenus avec un PBS comprenant du sérum de bélier hypophysectomisé sont illustrés par la figure 2 et dans le tableau 2 ci-dessous. La figure 2A représente une photographie de bâtonnets (« sticks ») après la mise en oeuvre du procédé de l'invention avec des solutions comprenant différente concentration de LH à savoir 0 ; 1 ; 2 ; 3,5 ; 5 et 10 ng/ml, les bâtonnets (« sticks ») ayant été préalablement recouvert avec une solution tampon PBS comprenant 50% en volume de sérum de bélier hypophysectomisé. La figure 2B représente un diagramme en bâton de l'intensité de la couleur obtenue sur les bâtonnets (« sticks ») en unités de densité fonction de la concentration de LH. Tel que montré sur la figure 2 A et sur le diagramme figure 2B, il est observé une très forte interférence non spécifique traduite par des bâtonnets (« sticks ») colorés avec la même intensité quel que soit la concentration de LH du plasma dosé.

L'interprétation visuelle (figure 2A) a été validée par la quantification de l'intensité colorée mesurée par scan (figure 2B et tableau 2). Il n'y a aucune différence significative entre les échantillons quel que soit leur concentration en LH.

**Tableau 2 : intensité de la couleur en fonction de la concentration de LH avec le tampon PBS comprenant du sérum de bélier hypophysectomisé**

| Concentration de LH (ng/ml) | Intensité de la couleur (unités de densité) | |
|---|---|---|
| | Moyenne | Ecart-type |
| 0 | 32,48 | 11,37 |
| 1 | 34,77 | 19,16 |
| 2 | 29 ,47 | 3,92 |
| 3,5 | 38,11 | 4,84 |
| 5 | 39,11 | 14,36 |
| 10 | 36,10 | 3,8 |

Tel que démontré dans cet exemple, la couleur apparue sur le bâtonnet (« stick ») est très forte quel que soit la concentration de LH dans les échantillons. Un fort signal non-spécifique a été obtenu avec le plasma sans LH (0 ng/ml) Tel que montré figure 2B et tableau 2: il n'y a aucune différence significative entre l'intensité de la couleur mesurée sur les différents bâtonnets (« sticks ») lorsque le tampon PBS comprenant du sérum de bélier hypophysectomisé est utilisé pour recouvrir la surface test et pour diluer l'anticorps anti-LH couplé à l'enzyme.

L'ensemble de ces résultats montrent donc clairement que le PBS seul ou comprenant du sérum de bélier hypophysectomisé ne sont pas des tampons adaptés et ne permettent pas une lecture visuelle.

Les résultats obtenus avec du PBS comprenant 20% en volume de SVF comme tampon de recouvrement (« surcoating ») et de préparation de l'anticorps couplé à l'enzyme, ici l'AC2 HRP sont illustrés sur la figure 3 A et 3B. La figure 3A représente une photographie de bâtonnets (« sticks ») après la mise en oeuvre du procédé de l'invention avec des solutions comprenant différentes concentrations de LH à savoir 0 ; 1 ; 2 ; 3,5 ; 5 et 10 ng/ml, les bâtonnets (« sticks ») ayant été préalablement recouverts avec une solution tampon PBS comprenant 20% en volume de sérum de veau foetal. La figure 3B représente un diagramme en bâton de l'intensité de la couleur obtenue sur les bâtonnets (« sticks ») en unités de densité en fonction de la concentration de LH. Tel que représenté dans la figure 3A et 3B et dans le tableau 3 ci-dessous, l'intensité de la couleur est proportionnelle à la concentration de LH. L'apparition d'une couleur bleue franche, détectable à l'oeil à partir de 2 ng/ml (figure 3A) est observée. Cette lecture visuelle est validée par une quantification de la couleur des bâtonnets (« sticks ») réalisée à l'aide d'un scanner (figure 3B et tableau 3). Une analyse statistique par test de Bonferroni a montré que l'apparition de la couleur est significative à partir de la concentration de 2 ng/ml qui représente le seuil définissant le début du pic pré-ovulatoire de LH (p<0,001).

**Tableau 3 : intensité de la couleur en fonction de la concentration de LH avec le tampon PBS comprenant du sérum de veau foetal à 20% en volume**

| Concentration de LH (ng/ml) | Intensité de la couleur (unités de densité) | |
|---|---|---|
| | Moyenne | Ecart-type |
| 0 | 4,58 | 0,59 |
| 1 | 12,02 | 1,62 |
| 2 | 17,5 | 4,01 |
| 3,5 | 23,48 | 2,17 |
| 5 | 28,10 | 2,81 |
| 10 | 32,9 | 5,88 |

La couleur apparue sur le bâtonnet (« stick ») est dépendante de la présence de LH, son intensité est proportionnelle à la concentration de LH. Le plasma sans LH (0 ng/ml) ne donne aucune couleur sur le bâtonnet (« stick »), témoignant d'une absence de signal non-spécifique.

Les résultats illustrés sur la figure 3, démontrent que les performances du bâtonnet (« stick ») sont très améliorées par l'utilisation du PBS comprenant 20% en volume de SVF.

En conclusion, le PBS comprenant du SVF permet une détection quantitative de la LH en fonction de sa concentration dans un échantillon de plasma.

### 2. Effet du pourcentage de SVF dans le PBS.

Dans cet exemple, différents pourcentages de SVF ont été testés.

Les bâtonnets (« sticks ») ont été préparés selon le procédé décrit dans l'exemple 1 ci-dessus. Le PBS a été préparé avec les pourcentages en volume de SVF suivants : 0 ; 5 ; 10 ; 20 ; 30 ; 40 et 50%. Deux plasmas bovin ont été utilisés comme échantillons à doser : l'un à 0 ng/ml l'autre à 7 ng/ml dans chacun des cas. L'intensité du signal coloré obtenu à 0 et 7 ng/ml de LH a été quantifiée dans chacun des cas. Pour chaque condition, le rapport entre les deux intensités a été calculé ; il représente le rapport du signal spécifique sur le bruit de fond.

Les résultats obtenus sont illustrés sur la figure 4 qui représente un diagramme en bâton du rapport intensité de la couleur avec une LH à 7 ng/ml (signal)/ intensité de la couleur avec une LH à 0 ng/ml (bruit de fond).

**Tableau 4 : résultats d'intensité lumineuse obtenue en fonction de la concentration de SVF**

| | | | | | | | |
|---|---|---|---|---|---|---|---|
| Pourcentage (en volume) de SVF | 0 | 5 | 10 | 20 | 30 | 40 | 50 |
| Intensité avec 0 ng/mL de LH | 37,58±4,41 | 13,51±1,03 | 11,25±1,74 | 9,97±1,28 | 9,94±0,15 | 9,41±1,09 | 8,28±0,63 |
| Intensité avec 7 ng/mL de LH | 37,17±5,97 | 29,72±3,02 | 26,85±1,28 | 26,32±5,01 | 25,59±3,59 | 23,93±3,06 | 22,55±3,08 |
| Rapport intensité à 7ng/ml /intensité à 0 ng/ml | 1,01 | 2,19 | 2,35 | 2,63 | 2,57 | 2,54 | 2,72 |

Tel que démontré sur la figure 4 et dans le tableau 4, l'ensemble des concentrations testées permettent d'obtenir un signal spécifique. En particulier, l'analyse statistique via la méthode d'analyse de variance à un facteur suivie d'un test de Bonferroni a montré que le pourcentage de 50% en volume de SVF permet d'obtenir le meilleur rapport signal spécifique / bruit de fondavec un seuil significatif à p<0,001. Ceux obtenus avec du PBS comprenant de 10 à 40% en volume de SVF sont également avantageux et sont très significativement différents des conditions 5% et 0% de SVF (**, p<0,01). La condition avec 5% en volume de SVF est significativement différente du PBS 0% en volume SVF (*, p<0,05).

Les résultats obtenus montrent que la détection de la LH avec du PBS comprenant de 20 à 50% de SVF est optimale avec un rapport signal/bruit de fond variant de 2,6 à 2,72 respectivement. La condition réalisée avec du PBS comprenant 50% en volume de SVF est la plus significative avec p<0,001. En-dessous du seuil de 20% en volume, le signal obtenu avec le plasma à 7ng/ml de LH est plus élevé mais en contrepartie, le signal non-spécifique augmente également.

Cet exemple démontre clairement que le procédé de l'invention permet une détection de la LH et une révélation visuelle de la détection.

### 3. Effet du substrat de l'enzyme.

Dans cet exemple, différents substrats de la peroxydase ont été évalués et comparés :
Tout d'abord le TMB membrane versus le TMB ELISA, puis le CN/ DAB, le DAB, le CN, l'OPD, l'ODN, l'AEC versus le TMB membrane de deux fournisseurs différents (KPL et Sigma).

### a) Effet de différents TMB. L'effet du TMB membrane commercialisé par la société KPL (référence 50-77-18) a tout d'abord été comparé avec celui du TMB Sure Blue (marque déposée) commercialisé par la société KPL (référence 52-00-00) utilisé pour les techniques ELISA.

Dans cet exemple, le procédé a été réalisé sur 4 échantillons de plasmas prélevés de façon sériée sur une même vache en période de pic préovulatoire de LH, en testant en parallèle chacun des révélateurs. Ces échantillons de plasmas ont été préalablement dosés quantitativement avec le kit de dosage LH DETECT (marque déposée). Leurs concentrations en LH étaient respectivement de 0 ; 2 ; 5 ; 10 ng/ml. Cette expérience a été répétée trois fois (n=3).

Les résultats obtenus sont représentés sur les figure 5A à C. La figure 5A représente une photographie de bâtonnets (« sticks ») obtenus après la mise en oeuvre du procédé de l'invention avec des plasmas comprenant différentes concentrations de LH à savoir 0 ; 1 ; 2 ; 5 et 10 ng/ml après révélation avec le TMB membrane La figure 5B représente une photographie de bâtonnets (« sticks ») après la mise en oeuvre du procédé de l'invention avec des plasmas comprenant différentes concentrations de LH à savoir 0, 1, 2, 5 et 10 ng/ml après révélation avec le TMB Elisa. La figure 5C représente un diagramme en bâton de l'intensité de la couleur obtenue sur les bâtonnets (« sticks ») en unités de densité en fonction de la concentration en LH. Les bâtons marqués avec un damier à grand carreaux représentent l'intensité de la couleur obtenue avec le TMB Elisa. Les bâtons marqués avec un damier à petits carreaux représentent l'intensité de la couleur obtenue avec le TMB Membrane.

Les résultats présentés sur la figure 5 démontrent très clairement que seul le TMB membrane donne un signal coloré sur le bâtonnet (« stick »). Ce signal coloré apparaît au seuil de 2 ng/ml de LH (figure 5A, figure 5C). A l'inverse aucune couleur n'est observée sur le bâtonnet (« stick ») révélé avec le TMB ELISA (figure 5B et figure 5C).

Le tableau 5 ci-dessous représente les résultats obtenus en fonction des substrats.

**Tableau 5 : intensité de la couleur en fonction de la concentration de LH et du substrat utilisé.**

| LH ng/ml | Intensité de la couleur (moyenne ± écart-type) | |
|---|---|---|
| | TMB Membrane | TMB ELISA |
| 0 | 4,76 ± 0,66 | 2,29 ± 0,79 |
| 2 | 16,84 ± 4,21 | 4,25 ± 1,77 |
| 5 | 22,88 ± 4,18 | 4,53 ± 1,74 |
| 10 | 27,81 ± 5,10 | 4,70 ± 1,83 |

Tel que démontré dans cet exemple, seuls les bâtonnets (« sticks ») révélés dans le TMB membrane présentent une couleur bleue très nette dont l'intensité évolue proportionnellement avec la concentration jusqu'à 10 ng/ml de LH. Avec l'échantillon sans LH, le bâtonnet (« stick ») reste blanc et ne présente aucun signal non-spécifique. Cette propriété permet avantageusement une lecture visuelle du test très simple.

La quantification de l'intensité de la couleur par scan (tableau 5 et figure 5C) a mis en évidence une augmentation très significative du signal avec le TMB Membrane dès la concentration de 2 ng/ml LH (*p<0,001*)*.* Cette couleur s'intensifie à 5 et 10 ng/ml de LH (tableau 5 et figure 5C). A l'inverse, la très faible amplitude observée avec le TMB ELISA n'est statistiquement pas significative.

Tel que démontré dans cet exemple, le TMB membrane est donc un révélateur efficace qui apporte les meilleures performances à la fois en termes de sensibilité du test (seuil à 2 ng/ml) et d'intensité de la réaction colorée (amplitude de 7 fois entre la couleur du plasma 0 et celle du plasma à 10ng/ml de LH).

### b) Test d'autres substrats spécifiques de la peroxydase : le CN/ DAB, le DAB, le CN, l'OPD par rapport au TMB membrane.

Dans cet exemple, les substrats CN/DAB (Thermo Scientific, référence 34000), DAB (Thermo Scientific, référence 34002), CN (Sigma, référence C6788) et OPD (Sigma, référence P5412)

Les bâtonnets (« sticks ») ont été préparés tel que décrit dans l'exemple 1 ci-dessus et ont été incubés dans un plasma bovin dosé à 19 ng/ml de LH. Le temps de contact dans chaque révélateur était de 15 minutes.

Chaque bâtonnet (« stick ») a été incubé dans un révélateur différent puis scanné afin de quantifier l'intensité de la couleur obtenue. Cette expérience a été répétée trois fois.

Les résultats sont représentés sur la figure 6. La figure 6 A représente une photographie des bâtonnets (« sticks ») révélés avec respectivement le tétrahydrochlorure de 3,3' - diaminobenzidine (DAB), le 4-chloro-1-naphtol (CN), le tétrahydrochlorure de 4-chloro-1-naphto / 3,3' - diaminobenzidine (CN/DAB), le OPD, le TMB Membrane commercialisé par la société KPL (TMB KPL) ou par la société Sigma (TMB Sigma). La figure 6B représente un diagramme en bâton de l'intensité de la couleur obtenue sur les bâtonnets (« sticks ») en unités de densité en fonction des différents substrats de l'enzyme indiqués ci-dessus.

Tel que représenté sur ces figures, seuls les deux TMB membrane donnent un signal coloré significatif, dont la valeur de quantification par scan est de 10 à 20 fois supérieure à celle des autres révélateurs utilisés.

Cette différence est très significative par rapport aux valeurs obtenues avec le CN/ DAB, le DAB, le CN, l'OPD (p<0,001).

L'ensemble de ces résultats indique que le TMB membrane permet d'obtenir un résultat visuel avec une meilleure sensibilité et spécificité du test de détection du pic pré ovulatoire de LH.

### c) Test de substrats spécifiques de la peroxydase par rapport au TMB membrane : le CN/ DAB, le DAB, le CN, l'OPD, l'ODN et l'AEC

Pour compléter l'étude comparative du point b) ci-dessus, les substrats précités ont été testés ainsi que deux autres substrats l'ODN et l'AEC (Sigma, références D3252 et A6926). Cette étude a été réalisée avec des bâtonnets (« sticks ») préparés dans des conditions extrêmes. Le recouvrement a été réalisé avec un anticorps de lapin anti-LH bovine fixé sur la surface test ((AC1 à 40µg/ml) et l'anticorps de cheval anti-LH ovine couplé à la péroxydase (AC2 HRP), a été également préparé à la concentration de 40µg/ml. Un plasma bovin à 19ng/ml a été utilisé. L'expérience a été répétée trois fois.

Les résultats sont représentés sur la figure 7A et B. La figure 7 A représente une photographie de bâtonnets (« sticks ») après la mise en oeuvre du procédé de l'invention avec des échantillons de plasma bovin comprenant une concentration de LH de 19 ng/ml révélé avec différents substrats de l'enzyme. Les substrats utilisés sont le tétrahydrochlorure de 3,3' - diaminobenzidine (DAB), le 4-chloro-1-naphtol (CN), le tétrahydrochlorure de 4-Chloro-1-naphto / 3,3' - diaminobenzidine (CN/DAB), le OPD (ortho-phénylène diamine), l'OND (3,3-diméthoxybenzidine,o-dianisidine), l'AEC (3-amino-9-éthylcarbazole), le TMB Membrane commercialisé par la société KPL (TMB KPL) ou par la société Sigma (TMB Sigma).

La figure 7B représente un diagramme en bâton de l'intensité de la couleur obtenue sur les bâtonnets (« sticks ») en unités de densité en fonction des différents substrats de l'enzyme : le tétrahydrochlorure de 3,3' - diaminobenzidine (DAB), le 4-chloro-1-naphtol (CN), le tétrahydrochlorure de 4-Chloro-1-naphto / 3,3' - diaminobenzidine (CN/DAB), le OPD (ortho-phénylène diamine), l'OND (3,3-Diméthoxybenzidine,o-dianisidine), l'AEC (3-amino-9-ethylcarbazole), le TMB Membrane commercialisé par la société KPL (TMB KPL) ou par la société Sigma (TMB Sigma).

**Tableau 7 : valeurs d'intensité obtenues en fonction du substrat.**

| | | | | | | | | |
|---|---|---|---|---|---|---|---|---|
| Substrat | CN/DAB | DAB | CN | TMB | TMB Sigma | OPD | ODN | AEC |
| Intensité | 20,3 | 3,7 | 2,6 | 80,6 | 96,1 | 0 | 2,6 | 4,0 |

Tel que montré dans les figures 7A et B et dans le tableau 7 ci-dessus, les deux TMB membrane permettent d'obtenir un signal coloré très élevé, c'est-à-dire entre 80 et 90 unités, quatre fois supérieur au signal obtenu avec le CN/DAB : 20 unités, ce dernier étant très difficilement détectable visuellement. Le DAB, le CN et l'OPD ne donnent aucun signal visible à l'oeil, soit entre 0 et 3,7 unités, le bâtonnet (« stick ») reste blanc après révélation. L'ODN et l'AEC donnent une légère coloration rosée détectable visuellement mais qui disparaît en quelques minutes. Pour cette raison, la quantification est impossible.

L'ensemble de ces résultats indiquent que les substrats CN/DAB, DAB, CN, OPD, ODN et AEC ne sont pas des révélateurs adaptés à l'utilisation du bâtonnet (« stick »). Même dans des conditions extrêmes de préparation des bâtonnets (« sticks »), utilisant des concentrations très élevées d'AC1 et d'AC2 HRP (40µg/ml), ces révélateurs ne développent qu'un signal nul ou très faible par rapport au TMB membrane. De plus, ces fortes concentrations d'anticorps anti-LH fixé sur la surface (AC1) et d'anticorps anti-LH couplé à la péroxydase (AC2 HRP) introduisent un signal non-spécifique élevé qui diminue beaucoup la sensibilité et la spécificité de détection.

En conclusion, comparativement aux autres substrats, le TMB membrane donne le meilleur rapport signal spécifique sur signal non-spécifique. L'intensité de la réaction colorée sur le bâtonnet (« stick ») et l'absence de bruit de fond en font le révélateur le plus avantageux, quel que soit le fournisseur du produit. Cette sensibilité optimale permet de détecter visuellement, sans ambiguïté, la présence de LH dans l'échantillon à doser par l'apparition d'un signal bleu sans aucune interférence non-spécifique.

### 4 Effet des conditions de préparation du TMB membrane.

Les inventeurs ont étudié l'intérêt d'une dilution du TMB membrane dans différents tampons afin de réduire au maximum le bruit de fond sans diminuer l'intensité du signal coloré,
Pour cela, trois paramètres ont été étudiés :
a) La dilution du TMB membrane dans différents tampons,
b) Le facteur de dilution du TMB membrane dans un tampon citrate, et
c) La molarité du tampon citrate utilisé.
Par chacune des séries, les résultats ont été comparés à ceux obtenus avec du TMB membrane pur. Les bâtonnets (« sticks ») ont été préparés dans les conditions de fabrication décrites dans l'exemple 1 ci-dessus.

### a) Préparation du TMB membrane dans différents tampons.

Afin d'évaluer l'impact de la composition du tampon de dilution du TMB membrane, trois tampons ont été utilisés parallèlement pour le diluer à50%:
- du PBS (K₂HPO₄/KH₂PO₄ 0,01M - NaCl 0,15M) pH 7,4 (commercialisé par VWR, références 26930.293, 26936.293, 27810.295),
- du tampon citrate (NaCH₃CO₂/CH₃COOH) 0,03M pH5 (commercialisé par VWR, référence 27652.298 et Carlo Erba Reagents, référence 302 002), et
- du tampon phosphate 0,01M (K₂HPO₄/KH₂PO₄) pH 7,4 (commercialisé par VWR, références 26930.293, 26936.293).

Deux échantillons de plasmas de vaches Holstein ont été utilisés : l'un à 0 ng/ml pour évaluer l'impact sur le bruit de fond et l'autre à 5ng/ml de LH pour évaluer l'impact sur l'intensité du signal coloré, ces deux paramètres conditionnant la sensibilité du test. Les résultats sont représentés sur la figure 8 et dans le tableau 8 ci-dessous.

La figure 8 représente un diagramme en bâton de l'intensité de la couleur obtenue sur les bâtonnets (« sticks ») en unités de densité en fonction de la concentration en LH : 0 ou 5 ng/ml et en fonction de la dilution à 50% du TMB membrane dans du PBS (K₂HPO₄/KH₂PO₄ 0,01M - NaCl 0,15M) pH 7,4 (bâtons avec les gros carrés); un tampon citrate (NaCH₃CO₂/CH₃COOH) 0,03M pH5 (bâtons hachurés horizontalement) ; du tampon phosphate 0,01M (K₂HPO₄/KH₂PO₄) pH 7,4 (bâtons hachurés verticalement).

**Tableau 8 : intensité de détection en fonction de la concentration en LH et en fonction du tampon de dilution du TMB membrane**

| Tampon | Pur | 1/2 PBS | 1/2 Tampon citrate | 1/2 Tampon phosphate |
|---|---|---|---|---|
| Intensité du signal avec 0 ng/ml de LH | 6,42 | 8,35 | 6,85 | 6,95 |
| Intensité du signal avec 5 ng/ml de LH | 37,77 | 29,98 | 43,45 | 44,5 |

Une étude statistique des intensités de couleur du bâtonnet (« stick ») obtenues a été effectuée avec le test de Bonferroni.

Les intensités du signal coloré obtenues avec le TMB dilué dans les trois tampons de dilution ne sont pas significativement différentes pour le plasma à 0 ng/ml de LH. Le tampon de dilution n'a donc pas d'influence significative sur le signal non-spécifique.

Avec le plasma 5 ng/ml, les intensités du signal coloré varient selon le tampon de dilution utilisé, mais ces différences ne sont pas significatives. Le TMB dilué à 50% dans le PBS présente néanmoins un signal coloré plus faible (29,98) comparativement au tampon citrate (43,45) et au tampon phosphate (44,5) qui donnent un signal coloré plus intense. Les inventeurs ont noté qu'il n'y a pas de différence significative entre les tampons citrate et phosphate : les intensités du signal coloré obtenu dans les deux cas sont quasiment égales avec une valeur un peu supérieure dans le cas d'une dilution avec le tampon citrate.

Les résultats et l'analyse statistique ont montré qu'avec le plasma à 0ng/ml, les intensités de couleur du bâtonnet (« stick ») obtenues ne sont pas statistiquement différentes les unes des autres.

Toutefois, les inventeurs ont démontré de manière surprenante que la préparation du TMB membrane dans 50% de tampon citrate 0,03M pH 4,5 apporte la meilleure performance du bâtonnet (« stick ») avec un signal zéro très bas et un signal à 5 ng/ml le plus élevé (meilleur rapport signal spécifique / bruit de fond). Ceci permet avantageusement de détecter le pic de LH avec une forte sensibilité et une forte spécificité et permet avantageusement une meilleure détection visuelle.

### b) Le facteur de dilution du TMB membrane dans le tampon citrate.

Différentes dilutions du TMB membrane dans du tampon citrate 0,03M ont été également évaluées afin d'optimiser la composition du révélateur et de minimiser les risques de signal non-spécifique en présence de concentrations très faibles de LH. Le TMB membrane a été dilué de 2 en 2 jusqu'au 1/16 en utilisant des bâtonnets (« sticks ») préparés selon les conditions standards de fabrication, c'est-à-dire selon le procédé décrit dans l'exemple 1 ci-dessus Un plasma de vache Holstein à 7,2ng/ml de LH a été utilisé comme échantillon.

Les résultats obtenus sont représentés sur la figure 9 et dans le tableau 9 ci-dessous.

La figure 9 représente un diagramme en bâton de l'intensité de la couleur obtenue sur les bâtonnets (« sticks ») en unités de densité en fonction de la dilution au 1/2, 1/4, 1/8 et 1/16 TMB membrane dans un tampon citrate un tampon citrate (NaCH₃CO₂/CH₃COOH) 0,03M pH5, ou sans dilution (pur).

Chaque expérience a été effectuée quatre fois. L'analyse statistique des résultats a été faite par analyse de variance suivie d'un test de Bonferroni.

**Tableau 9 : résultats de l'intensité de couleur en fonction de la dilution du tampon citrate**

| Dilution | Pur | 1/2 | 1/4 | 1/8 | 1/16 |
|---|---|---|---|---|---|
| Moyenne ± SEM | 22,27±1,68^{a} | 25,01±1,87^{a},^{b} | 21,39±1,38^{a} | 18,53±1,48^{c} | 11,93±1,95 |

Dans le tableau, a correspond à p<0,001 si comparé à une dilution au 1/16, b correspond à p<0,01 si comparé à une dilution au 1/8 et c correspond à p<0,01 si comparé à une dilution au 1/16.

Les résultats obtenus ont montré de façon surprenante que le TMB dilué au 1/2 dans du tampon citrate à 0,03M donne un signal coloré plus fort que celui obtenu avec le TMB pur ou dilué au 1/4. Cependant ces différences ne sont pas significatives. A partir d'une dilution au 1/4, la décroissance du signal est plus importante et aboutit à une valeur de 11,93 unités à la dilution 1/16. L'analyse statistique montre que seul le TMB membrane dilué au 1/2 est significativement différent de celui dilué au 1/8 et 1/16.

En conséquence et de façon surprenante, les inventeurs ont démontré que la dilution du TMB membrane au demi dans du tampon citrate permet d'obtenir une intensité du signal coloré plus forte en présence de LH et une intensité de signal coloré non-spécifique (bruit de fond) très basse c'est-à-dire inférieure à 5 unités de densité, valeur qui n'est plus détectable visuellement.

La dilution permet donc avantageusement d'augmenter la sensibilité de détection du procédé tout en permettant une meilleure détection visuelle.

### c) Effet de la molarité du tampon citrate utilisé pour diluer le TMB membrane.

Une étude de la concentration du tampon citrate a également été effectuée. Les concentrations testées étaient 0,003M, 0,03M, 0,3M et 3M. Les bâtonnets (« sticks ») utilisés ont été élaborés dans les conditions standards de fabrication, c'est-à-dire selon le procédé décrit dans l'exemple 1 ci-dessus. Un plasma de vache Holstein à 27ng/ml de LH a été utilisé comme échantillon.

Dans cet exemple, le TMB membrane a été dilué au 1/2 avec les différentes solutions tampons citrates précitées. Les résultats sont illustrés sur la figure 10 et dans le tableau 10 ci-dessous. La figure 10 représente un diagramme en bâton de l'intensité de la couleur obtenue sur les bâtonnets (« sticks ») en unités de densité en fonction de la concentration du tampon citrate en molaire.

**Tableau 10 : résultats de l'intensité de la couleur obtenue en fonction de la concentration Molaire du tampon citrate.**

| Molarité | 0,003 M | 0,03M | 0,3M | 3M |
|---|---|---|---|---|
| Intensité du signal | 17,9±1,3** | 20,1±4** | 15,1±1,9* | 0,2±0,3 |

L'analyse statistique des résultats a été faite par analyse de variance suivie d'un test de Bonferroni. Cette étude a montré que les signaux colorés des bâtonnets (« sticks ») obtenus aux molarités 0,003M, 0,03M et 0,3M ne sont statistiquement pas différents entre eux. A l'inverse, ces trois molarités sont significativement différentes du résultat obtenu avec un tampon citrate à 3M qui éteint tout signal coloré.

Ainsi, les inventeurs ont montré de manière surprenante que la molarité du tampon citrate a un effet sur la révélation du pic de LH. En particulier, les inventeurs ont montré de façon surprenante que la molarité 0,03M du tampon citrate permet avantageusement d'obtenir une forte coloration permettant une détection visuelle.

### 5 Comparaison de l'effet du SVF dans le tampon PBS par rapport à d'autres composants protéiques

L'effet de l'ajout de 50% en volume de SVF dans le PBS (K₂HPO₄/KH₂PO₄ 0,01M - NaCl 0,15M) pH 7,4) a été comparé à celui d'autres protéines ou sérum.
Pour cela, les 4 solutions suivantes ont été testées :
- PBS comprenant du SVF 50% en volume,
- PBS comprenant de la caséine 3% (30g/L),
- PBS comprenant de la BSA 4% (40g/L), et
- PBS comprenant du lait écrémé 5% (50g/L).

L'expérience a été répétée trois fois

Le SVF est le SVF commercialisé par Lonza sous la référence 14-801F, la caséine commercialisé par Sigma sous la référence C8654 l'albumine sérique bovine (BSA) commercialisée par PAA, sous la référence K45012 lait écrémé commercialisé par Régilait.

Les surfaces tests ont été préparées selon le procédé décrit dans l'exemple 1. Deux plasmas de vaches Holstein ont été utilisés comme échantillon : l'un à 0 ng/ml et l'autre à 7,7 ng/ml. L'intensité du signal coloré obtenu a été quantifiée dans chacun des cas selon le procédé décrit ci-dessus. Les résultats sont représentés dans la figure 11 et dans le tableau 11 ci-dessous. La figure 11 représente un diagramme en bâton de l'intensité de la couleur obtenue sur les bâtonnets (« sticks ») en unités de densité en fonction de la concentration en LH : 0 ou 7,7ng/ml et en fonction du composant protéique présent dans le tampon phosphate salin, à savoir du sérum de veau foetal à 50% en volume (bâtons avec des petits carrés), de la caséine à 3 % (30g/L) (bâtons remplis avec des gros carrés), de l'albumine sérique bovine (BSA) à 4% (40g/L) (bâtons hachurés en horizontal), ou du lait écrémé à 5% (50g/L) (bâtons hachurés horizontaux).

**Tableau 11 : résultats de l'intensité de la couleur obtenue en fonction du composant protéique**

| PBS comprenant en volume | SVF 50% | Caséine 3% | BSA 4% | Lait écrémé 5% |
|---|---|---|---|---|
| Signal à 0 ng/ml LH | 5,16 ± 0,8 | 25,22 ± 1,36 | 4,39 ± 1,09 | 12,51 ± 3,64 |
| Signal à 7,7 ng/ml LH | 28,11 ± 0,41 | 35,65 ± 0,49 | 20,89 ± 1,38 | 27,29 ± 1,49 |
| Rapport signal / bruit de fond | 5,45 | 1,41 | 4,75 | 2,18 |

Tel que démontré sur la figure 11 et dans le tableau 11 ci-dessus, avec du PBS comprenant 50% en volume de SVF ou 4% (40g/L) de BSA une très faible coloration, non visuelle est obtenue avec le plasma à 0ng/ml de LH démontrant avantageusement l'absence de signal non-spécifique.

A l'inverse, une coloration plus élevée de la surface et détectable visuellement est obtenue avec du PBS comprenant 5% (50g/L) de lait écrémé (12,51) montrant un signal non-spécifique désavantageux. Le PBS comprenant 3% (30g/L) de caséine est encore moins favorable, donnant un signal non-spécifique très élevé (25,22).

Dans tous les cas, les valeurs obtenues avec le plasma 7,7ng/ml de LH sont significativement différentes de l'intensité obtenue avec le plasma 0ng/ml de LH: pour les tampons PBS-SVF 50% en volume, PBS-BSA 4% (40g/L) et PBS-lait 5% (50g/L) (***, p<0,001) et pour le PBS-caséine 3% (30g/L) (**, p<0,01).

L'impact des différents milieux a été précisé en calculant le rapport signal (à 7,7ng/ml) sur bruit de fond (à 0ng/ml). Ce rapport varie selon le tampon utilisé, il est le plus favorable avec le PBS - SVF 50% en volume (5,45) et le moins favorable avec le PBS - caséine 3% (30g/L) (1,41).

En conclusion, ces résultats démontrent l'importance des composants protéiques rajoutés dans le tampon de recouvrement de la surface test et de préparation de l'anticorps anti-LH couplé à une enzyme, dans cet exemple l'anticorps anti-LH couplé à la péroxydase (AC2 HRP).

Tel que démontré dans cet exemple, selon le tampon utilisé, les performances du bâtonnet (« stick ») sont en effet très différentes. Le meilleur résultat est obtenu avec le PBS comprenant du SVF, en particulier 50% en volume de SVF.

### 6 Essais comparatifs avec différentes enzymes

D'autres enzymes de couplage ont été testées afin d'évaluer leur fonctionnalité dans le procédé de l'invention et la trousse telle que fabriquée selon le procédé décrit dans l'exemple 5
Trois enzymes ont ainsi été comparées avec la peroxydase :
- la glucose oxydase,
- la beta-galactosidase, et
- la phosphatase alcaline.

La glucose oxydase utilisée était la glucose oxydase commercialisée par Sigma Aldrich provenant d'*aspergillus niger* sous la référence catalogue Réf : G7141 sous forme de poudre. La poudre a été pesée et resuspendue à 1 mg/ml dans1 ml tampon carbonate-bicarbonate 0,1M (NaHCO₃/Na₂CO₃ 0,1M pH 9,6) commercialisé respectivement par Prolabo sous la référence 27778.293 et par VWR sous la référence 27771.290).

Le recouvrement de la surface test (mise en contact), des ailettes du bâtonnet (« stick ») a été réalisé directement en l'incubant dans la solution à 1mg/ml pendant une heure à 37°C, puis pendant une heure à 4°C. Les bâtonnets (« sticks ») ont été lavés pendant 10 secondes dans un récipient comprenant du PBS (K₂HPO₄/KH₂PO₄ 0,01M - NaCl 0,15M) pH 7, (VWR, ref. 26930.293, 26936.293, 27810.295) et plongés pendant 15 minutes dans la solution de substrat comprenant:
7,5g D-glucose,
0,1g PMS (phénazine méthosulfate), et
0,5g NBT (Nitro-Blue-tétrazolium),
dissous dans 1 litre du tampon 0,1M Na₂HPO₄ pH 6,9.

La figure 12A représente la photographie de la surface test (ailettes du bâtonnet (« stick »)) obtenue après révélation et montre qu'aucune couleur n'apparaît ni sur le bâtonnet (« stick »), qui reste blanc, ni dans la solution de substrat (figure 12 B).

Un contrôle positif a été réalisé en rajoutant 25µl de glucose oxydase à 1mg/ml dans 250µl de substrat ; dans ce cas, un précipité bleu intense apparaît instantanément. Ces résultats démontrent que la glucose oxydase est active en solution (figure 12 C) mais ne réagit pas avec son substrat quand elle est adsorbée sur la surface du bâtonnet (« stick ») (figure 12 C).

La beta-galactosidase utilisée est commercialisée par Sigma Aldrich provenant d'Escherichia. coli sous la référence catalogue Réf: G5635 sous forme de poudre. Elle a été préparée à 1 mg/ml dans 1 ml de tampon carbonate-bicarbonate (NaHCO₃/Na₂CO₃ 0,1M pH 9,6) poudres commercialisées respectivement par Prolabo sous la référence 27778.293 et par VWR sous la référence 27771.290).

Le recouvrement de la surface test (mise en contact), des ailettes du bâtonnet (« stick ») a été réalisé par incubation avec une solution à 50µg/ml pendant une heure à 37°C, puis pendant une heure à 4°C.

Les bâtonnets (« sticks ») ont été lavés 10 secondes dans un récipient comprenant du PBS (K₂HPO₄/KH₂PO₄ 0,01M - NaCl 0,15M, pH 7,4) (poudres commercialisées par VWR, ref. 26930.293, 26936.293, 27810.295)et plongés pendant 18 heures à 37°C dans la solution de substrat comprenant: X-Gal (commercialisé par la société Uptima/Interchim sous forme de poudre référence catalogue Réf : UP40534M) dilué à 4 mg/ml dans 1 ml de DMSO (Sigma, ref D8418) puis à 1 mg/ml dans 1 ml de PBS (K₂HPO₄/KH₂PO₄ 0,01M - NaCl 0,15M, pH 7,4) Une autre préparation de substrat X-Gal a également été testée : une solution mère préparée à 40 mg/ml dans 1 ml de DMSO (Sigma, ref D8418) a été diluée à 1 mg/ml dans 1 ml de PBS (K₂HPO₄/KH₂PO₄ 0,01M - NaCl 0,15M, pH 7,4) (poudres commercialisées par VWR, références 26930.293, 26936.293, 27810.295 additionné de 3 mM ferrocyanide de potassium (1,27 g/l), 3 mM ferricyanide de potassium (0,99 g/l) (Sigma, ref. HT201 et 702587)

Les résultats ont montré que dans les deux cas, aucune couleur n'apparaît ni sur le bâtonnet (« stick ») (surface test) qui est resté blanc (figure 13 A), ni dans le substrat resté en contact avec le bâtonnet (« stick ») 18 heures à 37°C (figure 13 B).

Un contrôle positif réalisé en rajoutant 12,5µl de beta-galactosidase à 1mg/ml dans 250µl de substrat présente un précipité bleu intense qui apparaît instantanément (figure 13C).

Ces résultats démontrent que la beta-galactosidase est active en solution mais ne réagit pas avec son substrat quand elle est adsorbée sur la surface du bâtonnet (« stick »).

La phosphatase alcaline utilisée est commercialisée par Sigma Aldrich sous la référence catalogue P0114, sous forme liquide à 19mg/ml. Cette solution a été adsorbée sur la surface test, ailettes du bâtonnet (« stick ») à la concentration de 50µg/ml par incubation pendant une heure à 37°C puis pendant une heure à 4°C.

Les bâtonnets (« sticks ») ont été lavés 10 secondes dans du PBS (K₂HPO₄/KH₂PO₄ 0,01M - NaCl 0,15M, pH 7,4) (poudres commercialisées par VWR, ref. 26930.293, 26936.293, 27810.295) et plongés pendant 30 minutes dans une solution de substrat BCIP/NBT commercialisé par la société Uptima/Interchim sous la référence catalogue UP09985.

Les résultats illustrés sur la figure 14 montrent qu'une couleur bleue apparaît dans ce cas, sur le bâtonnet (« stick »), démontrant que la phosphatase alcaline directement adsorbée sur le bâtonnet (« stick ») réagit avec son substrat et développe un signal coloré.

Une deuxième expérience a été réalisée avec des bâtonnets (« sticks ») dont les ailettes ont été mises en contact et sur lesquelles l'anticorps polyclonal anti-LH bovine fait chez le lapin à 20µg/ml a été fixé. L'anticorps a été produit par le prestataire Eurogentec (Belgique) selon son procédé «Standard anti-protein packages 28-day Speedy in Rabbit» (marque de commerce) puis les ailettes (surface test) ont été mises en contact et recouvertes avec une solution de PBS (K₂HPO₄/KH₂PO₄ 0,01M - NaCl 0,15M, pH 7,4) (poudres commercialisées par VWR, ref. 26930.293, 26936.293, 27810.295) comprenant 50% (en volume) de SVF (sérum de veau foetal commercialisé par Lonza, référence 14-801F) selon le protocole de fabrication décrit dans l'exemple 1. Après 15 minutes d'incubation dans un plasma de vache Holstein dosé à 7ng/ml de LH ou dans une solution sans LH (0 ng/ml), les bâtonnets (« sticks ») ont été incubés 15 minutes dans une solution d'anticorps anti-LH non couplé (AC2 non couplé) qui étaient des anticorps polyclonaux anti-LH fait chez le cheval selon le procédé décrit dans l'exemple 1 préparé à 10µg/ml dans 500µl de PBS-SVF à 50% (en volume) (voir composition ci-dessus) Après lavage dans 50 ml de PBS (K₂HPO₄/KH₂PO₄ 0,01M - NaCl 0,15M, pH 7,4), les bâtonnets (« sticks ») ont été essorés puis incubés 15 minutes:
- soit dans une solution d' anticorps polyclonal anti-IgG de cheval, fait chez le lapin conjugué à la phosphatase alcaline (AC3-PA), commercialisé par la société Jackson Laboratories sous la référence 308-035-003, et préparé au 1/1250^{ème} dans du PBS-SVF à 50% (en volume) (voir composition ci-dessus). Après lavage dans 50 ml de PBS (K₂HPO₄/KH₂PO₄ 0,01M - NaCl 0,15M, pH 7,4), les bâtonnets (« sticks ») ont été révélés dans une solution de substrat BCIP/NBT commercialisé par la société Uptima/Interchim sous la référence catalogue UP09985.
- soit dans une solution d'anticorps polyclonal anti-IgG de cheval, fait chez le lapin conjugué à la peroxydase (AC3-HRP), commercialisé par la société Jackson Laboratories sous la référence 308-035-003, et préparé au 1/1250^{ème} dans du PBS-SVF à 50% (en volume) (voir composition ci-dessus). Après lavage dans 50 ml de PBS (K₂HPO₄/KH₂PO₄ 0,01M - NaCl 0,15M, pH 7,4), les bâtonnets (« sticks ») ont été révélés dans une solution de substrat TMB membrane commercialisé par la société KPL.

La figure 15 est une photographie représentant les résultats obtenus avec les deux séries de bâtonnets (« sticks ») et montre que le signal obtenu avec l'AC3 conjugué à la phosphatase alcaline (Ac3-PA) est très faible (figure 15, Ac3PA, 7 ng/ml LH) avec cependant un signal non-spécifique élevé (figure 15, Ac3PA, 0 ng/ml LH). A l'inverse, le signal coloré obtenu avec l'AC3 conjugué à la peroxydase (figure 15, Ac3HRP, 7 ng/ml LH) est deux fois plus intense et aucun bruit de fond n'est observé (figure 15, Ac3PA, 0 ng/ml LH).

Les valeurs d'intensités obtenues sont indiquées dans le tableau 12 ci-dessous.

**Tableau 12 : intensité de la coloration obtenue en fonction de l'enzyme couplée**

| Phosphatase alcaline | | Peroxydase | |
|---|---|---|---|
| Plasma 0ng/ml LH | Plasma 7ng/ml LH | Plasma 0ng/ml LH | Plasma 7ng/ml LH |
| 4,31 | 8,15 | 0,8 | 16,6 |

Cet exemple démontre clairement que la peroxydase est l'enzyme la plus fonctionnelle, la plus efficace et la mieux appropriée pour la mise en oeuvre du procédé de détection du pic de LH, d'une grande sensibilité et d'une lecture visuelle facile, interprétable sans ambiguïté.

### f) Essais comparatifs avec différents anticorps anti-LH

Le procédé et la trousse tels que décrits dans les exemples 1 et 5 ont été mis en oeuvre en utilisant soit un anticorps anti-LH différent (AC1 ou AC2) soit en utilisant deux anticorps anti-LH différents (AC1 et AC2). Trois conditions ont été évaluées :
- anticorps fixé sur la surface test (AC1) identique à celui de l'exemple 1 et anticorps couplé à l'enzyme (AC2 HRP) différent,
- anticorps fixé sur la surface test différent et anticorps couplé à l'enzyme identique à celui de l'exemple 1, et
- anticorps fixé sur la surface test et anticorps couplé à l'enzyme, tous deux différents par rapport à l'exemple 1.

Dans le premier cas, les bâtonnets (« sticks ») (surface test) ont tous été recouverts avec l'anticorps utilisé dans l'exemple 1 à savoir l'anticorps polyclonal anti-LH bovine fait chez le lapin, préparé à 20µg/ml. Quatre plasmas de vache Holstein à 0 ; 1 ; 3,5 et 5ng/ml de LH respectivement ont été utilisés comme échantillons. L'expérience a été répétée 3 fois.

Deux anticorps couplés à la peroxydase, ont été testés: l'anticorps polyclonal utilisé dans l'exemple 1 à savoir l'anticorps polyclonal anti-LH ovine fait chez le cheval (AC2 HRP), il s'agit de l'anticorps de l'exemple 1 , et un anticorps polyclonal anti-LH porcine fait chez le lapin (AC2 HRP nouveau), il s'agit de l'anticorps produit, par exemple par immunisation de lapins selon le procédé décrit par exemple dans la référence bibliographique « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ;266 :2291-2294 [5]. Il s'agissait du procédé comprenant par exemple trois injections de 200 µg de LH porcine purifiée commercialisée par la société Tucker Endocrine Research Institute LLC TUENRE (Atlanta, USA), qui ont été réalisées toutes les deux semaines, suivi de dix rappels avec 100µg de LH porcine purifiée effectués toutes les cinq semaines. Les saignées ont été réalisées six et neuf jours après chaque rappel et40.ml de sang ont été récupérés. A partir des échantillons de sang les anticorps de lapin ont été purifiés par chromatographie d'affinité sur gel de Protéine A sépharose selon le procédé décrit dans « Techniques Immuno-enzymatiques » Thérèse Ternynck et Stratis Avrameas, éditions INSERM, 1987 [1] Le couplage de l'anticorps a été réalisé selon le procédé décrit dans « Techniques Immuno-enzymatiques » Thérèse Ternynck et Stratis Avrameas, éditions INSERM, 1987 [1]. Les deux anticorps couplés à la péroxydase ont été préparés à la concentration de 10 µg/ml dans du PBS-SVF à 50% (en volume) (K₂HPO₄/KH₂PO₄ 0,01M - NaCl 0,15M, pH 7,4) (poudres commercialisées par VWR, ref. 26930.293, 26936.293, 27810.295) comprenant 50% (en volume) de SVF (sérum de veau foetal commercialisé par Lonza, référence 14-801F). Le révélateur utilisé était du TMB membrane commercialisé par KPL sous la référence 50-77-18 dilué au demi dans du tampon citrate (NaCH₃CO₂/CH₃COOH 0,03M, pH 5, poudres commercialisées par VWR, référence 27652.298 et Carlo Erba Reagents, référence 302 002).

Les résultats obtenus sont illustrés sur la figure 16 et sur le tableau 13 ci-dessous. La figure 16 représente une photographie de bâtonnets (« sticks ») après la mise en oeuvre du procédé de l'invention avec des échantillons de plasmas de vache comprenant une concentration de LH de 0 ; 1 ; 3,5 et 10 ng/ml en fonction de l'anticorps couplé à la péroxydase à savoir l'anticorps couplé identique à celui de l'exemple 1 (AC2 HRP) indiqué AC2 HRP classique ou un anticorps couplé différent tel que présenté ci-dessus indiqué AC2 HRP nouveau.

**Tableau 13 : intensité de la coloration obtenue en fonction de l'anticorps couplé et de la concentration de LH des plasmas**

| AC2 HRP | Anticorps couplé identique à celui de l'exemple 1 (AC2 HRP) | | | | Anticorps couplé différent (AC2 HRP nouveau) | | | |
|---|---|---|---|---|---|---|---|---|
| LH (ng/ml) | 0 | 1 | 3,5 | 10 | 0 | 1 | 3,5 | 10 |
| Intensité du signal | 5,77 | 8 | 27,45 | 32,9 | 7,87 | 15,73 | 37,78 | 55,16 |

Quel que soit l'anticorps couplé à la péroxydase utilisé, il a été observé un signal coloré proportionnel à la concentration en LH des plasmas (figure 16 et tableau 13 ci-dessus). A la concentration de 10µg/ml, le nouvel anticorps anti-LH couplé à la péroxydase (AC2 HRP nouveau) donne une intensité de couleur plus élevée mais génère un signal non-spécifique léger, intensité du signal de 7,87 unités, visible à l'oeil. A l'inverse, l'AC2 HRP utilisé dans l'exemple 1 n'induit aucun bruit de fond détectable à l'oeil avec une intensité du signal de 5 unités.

Dans un deuxième cas, deux anticorps polyclonaux anti-LH du commerce, sous forme d'immun sérums ont été utilisés afin d'être fixés sur la surface test. Il s'agit de l'anticorps anti-LH bovine commercialisé par la société US Biologicals Cat N°L7500-04P, lot N°L11010677, immun sérum dilué 400 000 fois, et de l'anticorps anti-LH porcine commercialisé par la société US Biologicals Cat N°L7500-02H, Lot N°L11100302, immun sérum dilué 100 fois.

Deux plasmas de vache ou de truie à 0 et 5 ng/ml de LH obtenus après centrifugation de prises de sang, ont été utilisés comme échantillons. L'anticorps anti-LH couplé était celui utilisé dans l'exemple 1 précité (AC2 HRP) et a été préparé à 10µg/ml dans du PBS-SVF à 50% (K₂HPO₄/KH₂PO₄ 0,01M - NaCl 0,15M, pH 7,4) (poudres commercialisées par VWR, ref. 26930.293, 26936.293, 27810.295) comprenant 50% (en volume) de SVF (sérum de veau foetal commercialisé par Lonza, référence 14-801F). Le révélateur utilisé était du TMB membrane commercialisé par KPL sous la référence 50-77-18dilué au demi dans du tampon citrate (NaCH₃CO₂/CH₃COOH 0,03M, pH5, (VWR, référence 27652.298 et Carlo Erba Reagents, référence 302 002).

Les résultats sont illustrés sur la figure 17 et sur le tableau 14 ci-dessous. La figure 17 représente une photographie de bâtonnets (« sticks ») après la mise en oeuvre du procédé de l'invention avec des échantillons de plasma bovin comprenant une concentration de LH de 0 ou 5 ng/ml en fonction de l'anticorps fixé sur la surface test, à savoir un anticorps de lapin anti-LH bovine (AC 1 : Anti-LH bovine) ou un anticorps de lapin anti-LH porcine (AC1 : Anti-LH porcine).

**Tableau 14 : intensité obtenue en fonction de l'anticorps 1 anti-LH**

| AC1 commerciaux | anti-LH bovine | | anti-LH porcine | |
|---|---|---|---|---|
| LH (ng/ml) | 0 | 5 | 0 | 5 |
| Intensité du signal | 5,88 | 24,18 | 5,4 | 11,23 |

Tel que démontré, aucune intensité de coloration visuelle (inférieur à 6) n'a été obtenue avec les échantillons sans LH et donc ne produit pas de signal non-spécifique. Par ailleurs, une coloration visuelle de la surface test a été obtenue avec les deux anticorps lors du test avec un plasma comprenant 5 ng/ml de LH. Toutefois, l'intensité était plus faible, mais tout à fait visible c'est-à-dire supérieure à 6 unités de densitométrie dans le cas de l'AC1 anti-LH porcine.

Enfin, le bâtonnet (« stick ») a été préparé en utilisant un anticorps fixé et un anticorps couplé à une enzyme différents de l'exemple 1: l'anticorps fixé AC1 était spécifique de la LH bovine, il s'agissait de l'anticorps commercialisé par la société US Biologicals Cat N°L7500-04P, lot N°L11010677, immun sérum dilué 400 000 fois et l'anticorps couplé à l'enzyme, la péroxydase, était un anticorps anti-LH bovine fait chez le lapin et couplé à la peroxydase (AC2 HRP nouveau) selon le procédé décrit dans « Techniques Immuno-enzymatiques » Thérèse Ternynck et Stratis Avrameas, (éditions INSERM, 1987) [1]. L'anticorps a été produit par le prestataire Eurogentec (Belgique) selon son procédé «Standard anti-protein packages 28-day Speedy in Rabbit» (marque de commerce) à partir de LH bovine purifiée. L'anticorps couplé à la péroxydase utilisé dans l'exemple 1 (AC2 HRP classique) a également été utilisé pour la comparaison.

La figure 18 représente une photographie de bâtonnets (« sticks ») après la mise en oeuvre du procédé de l'invention avec des échantillons de plasmas bovins comprenant une concentration de 0 ou 6,2 ng/ml de LH en fonction de l'anticorps anti-LH couplé à l'enzyme, à savoir un anticorps anti-LH ovine fait chez le cheval (AC2 HRP classique) et un anticorps polyclonal anti-LH porcine fait chez le lapin (AC2 HRP nouveau).

**Tableau 15: intensité obtenue en fonction de l'anticorps anti-LH couplé à la péroxydase**

| | AC2 HRP classique | | AC2 HRP nouveau | |
|---|---|---|---|---|
| LH (ng/ml) | 0 | 6,2 | 0 | 6,2 |
| Intensité du signal | 4,34 | 24,76 | 2,45 | 54,83 |

L'ensemble de ces résultats, répétés sur trois expériences indépendantes, démontrent donc clairement que le procédé de l'invention utilisant un tampon PBS comprenant du SVF, pour sa mise en contact avec la surface test sur laquelle est fixée un anticorps anti-LH et pour la préparation de l'anticorps anti-LH couplé à une enzyme, et, l'utilisation du TMB membrane comme substrat, permet avantageusement d'obtenir un test sensible et fonctionnel, quel que soit les anticorps utilisés.

Dans les deux séries d'anticorps couplés à la péroxydase (AC2 HRP), il a été obtenu un signal coloré intense, équivalent, avec le plasma à 6,2 ng/ml (tableau 15, figure 18) et aucune couleur (aucun bruit de fond) avec le plasma négatif à 0 ng/ml de LH (tableau 15, figure 18). La lecture visuelle des résultats des tests est donc la même quel que soit la combinaison des anticorps utilisés.

Cet exemple démontre donc clairement que le procédé de l'invention permet avantageusement la détection visuelle du pic de LH avec une sensibilité et une spécificité accrues.

En outre, la trousse pour la mise en ouvre du procédé permet d'obtenir un test fiable et sensible.

### Exemple 3 : mise en oeuvre du procédé de détection du pic de LH chez le bovin dans différents milieux biologiques

Dans cet exemple, le procédé de détection du pic de LH a été utilisé dans différents échantillons biologiques : le sang, le plasma et le mucus vaginal obtenus à partir de l'espèce bovine. Les essais ont été effectués à partir de vaches de différentes races : Holstein, Montbéliarde, Charolaise et Blonde d'Aquitaine.

Dans chaque essai, les résultats obtenus avec le procédé de l'invention ont été systématiquement corrélés avec un dosage ELISA quantitatif réalisé sur les mêmes échantillons à l'aide du kit LH DETECT (marque déposée) (ReproPharm SA, France). Pour la présentation des résultats ci-dessous, la lecture visuelle des tests a été validée par une quantification densitométrique de la couleur des bâtonnets (« sticks »). Pour cela, les bâtonnets (« sticks ») sont scannés à l'aide d'un Scanner EPSON (Perfection 1200 PHOTO) puis l'intensité de la couleur obtenue sur chacun d'eux est quantifiée par densitométrie avec le logiciel « Scion Image » (Scion Incorporation). Cette quantification est exprimée en unités de densité.

La collecte du sang a été préalablement réalisée soit à l'aide d'une prise de sang sous vacutainer hépariné au niveau de la veine caudale ou jugulaire, soit par scarification au niveau de l'oreille. Lorsque le prélèvement a été réalisé au niveau de l'oreille, la vache a été bloquée au conardis et sa tête maintenue en position latérale avec un licol, puis, sur la face externe de l'oreille, le prélèvement a été effectué après la division de la veine inférieure. La zone pour le prélèvement a été préalablement nettoyée à l'alcool et frotté pour faire gonfler les veines. Un stylet de 5mm de largeur (Goldenrod Animal Lancet, fabriquant MEDIPOINT) a été disposé perpendiculairement à la peau tout en tenant l'oreille. Une pression en amont de la veine a été effectuée afin d'augmenter son volume. Un tube a été ensuite positionné sous l'entaille et le sang laissé couler dedans. Le saignement a été stoppé par pression au niveau de la zone de prélèvement.

Dans le cas d'une scarification, la récupération de 4 à 5 gouttes de sang a été effectuée dans le tube n°1 de la trousse telle que présenté dans l'exemple 5 ci-dessous. Elle a été réalisée à l'aide d'un stylet de 5mm de largeur (Goldenrod Animal Lancet, fabriquant MEDIPOINT).

### a) Exemple de détection du pic préovulatoire de LH dans le sang chez la vache Holstein.

L'exemple présente les résultats obtenus en ferme, sur une vache Holstein représentative, au 19^{ème} jour de son cycle sexuel, en fin de phase lutéale. Les prises de sang ont été effectuées toutes les 30 minutes à partir d'une injection de 2 ml d'un analogue du GnRH, la Cystoréline (gonadoliberine 0,05 mg/ml, CEVA Santé Animale).

Sur chaque échantillon, la détection du pic de LH a été réalisée à l'aide de la trousse de l'exemple 5 ci-dessous selon son mode opératoire suivant :
Le prélèvement de l'échantillon a été préalablement réalisé tel que décrit ci-dessus puis les tubes de la trousse de l'exemple 5 ont été sortis du congélateur 30 min avant utilisation.

Le premier tube (tube 1) a été pris (bouchon sans pastille de couleur) et le bâtonnet (« stick ») sorti du tube. Le bâtonnet (« stick ») a été posé à l'envers en équilibre sur le bouchon afin de ne pas souiller l'extrémité du bâtonnet (« stick »).

Le prélèvement de sang a été réalisé tel que décrit ci-dessus. Environ 5 gouttes de sang ont été collectées dans le tube 1 jusqu'au niveau inférieur de l'étiquette puis le bâtonnet (« stick ») a été revissé sur ce tube. L'ensemble a été laissé incuber 15 min au minimum à température ambiante à savoir 25°C en position verticale, l'extrémité du bâtonnet (« stick ») trempant dans le sang.

Le bâtonnet (« stick ») a été ensuite sorti et secoué pour éliminer l'excès de sang avant de le plonger dans le pot de lavage (pot à bouchon BLANC) comprenant dans 50 ml de PBS (K₂HPO₄/KH₂PO₄ 0,01M - NaCl 0,15M, pH 7,4) et lavé énergiquement dans le liquide en faisant des cercles, pendant 10 secondes. Le bâtonnet (« stick ») a été secoué après le lavage afin d'éliminer l'excès de liquide.

Le second tube comprenant le réactif c'est-à-dire l'anticorps anti-LH couplé à la péroxydase (tube 2) (bouchon marqué avec une pastille JAUNE), a été ouvert par dévissage du bouchon et le bâtonnet (« stick ») a été vissé à sa place. L'ensemble a été laissé à incuber 15 min au minimum à température ambiante à savoir 25°C, en position verticale (l'extrémité du bâtonnet (« stick ») trempant dans le réactif).

Le bâtonnet (« stick ») a été sorti puis secoué afin d'éliminer l'excès de réactif avant de le plonger dans le pot de lavage comprenant 50 ml de PBS (K₂HPO₄/KH₂PO₄ 0,01M - NaCl 0,15M, pH 7,4) (pot à bouchon BLANC) et lavé pendant 10 secondes en le remuant énergiquement dans le liquide en faisant des cercles. Le bâtonnet (« stick ») a été secoué après le lavage afin d'éliminer l'excès de liquide.

Le troisième tube comprenant un révélateur (tube 3) a été ouvert par dévissage du bouchon et le bâtonnet (« stick ») a été vissé à sa place. L'ensemble a été laissé incuber 5 min au minimum à température ambiante en position verticale (l'extrémité du bâtonnet (« stick ») trempant dans le révélateur).

Le bâtonnet (« stick ») a été dévissé et la lecture et l'interprétation ont été effectuées comme suit avec en outre la mesure de l'intensité de la couleur tel que décrit précédemment et un dosage ELISA quantitatif réalisé sur les mêmes échantillons à l'aide du kit LH DETECT (marque déposée). Si l'extrémité du bâtonnet (« stick ») est BLEUE : le test est POSITIF et indique la présence d'un pic de LH. La vache doit être inséminée environ 12 heures plus tard. Si l'extrémité du bâtonnet (« stick ») est blanche : le test est NEGATIF et témoigne d'une absence de pic de LH.

Les résultats obtenus sont illustrés sur la figure 19. La figure 19 A représente des photographies de bâtonnets (« sticks ») obtenus après la mise en oeuvre du procédé sur les différents prélèvements de sang réalisés sur la vache. La figure 19B représente un diagramme de la concentration en LH en ng/ml en fonction de l'heure des prélèvements de sang.

Les mêmes prélèvements de sang ont été analysés qualitativement avec la présente trousse (figure 19A) et quantitativement avec LH DETECT (marque déposée) (figure 19B). Les valeurs du signal coloré des bâtonnets (« sticks ») ainsi que les concentrations de LH mesurées avec LH DETECT (marque déposée) sont indiquées dans le tableau 15.

**Tableau 15: valeurs du signal coloré des bâtonnets (« sticks ») et de la concentration de LH mesurée avec LH DETECT (marque déposée)**

| | | | | | | | | |
|---|---|---|---|---|---|---|---|---|
| Heures de prélèvement | 8H30 | 9H30 | 10H | 10H30 | 11H | 11H30 | 12H | 13H |
| Intensité du signal | 4,8 | 26,3 | 34,2 | 38,9 | 42,1 | 38,7 | 29,3 | 21,2 |
| Concentration de LH (ng/ml) | 0,2 | 7,1 | 11,0 | 15,3 | 19,2 | 13,0 | 9,0 | 3,7 |

Tel que démontré sur la figure 19 et sur le tableau 15, une augmentation parallèle de la concentration et du signal coloré des bâtonnets (« sticks ») de 9H30 à 11H puis une diminution jusqu'à 13H ont été observées. Avant l'injection de Cystoréline, à 8H30, la concentration est très faible, et corrèle très bien avec le bâtonnet (« stick ») resté blanc. Les valeurs des concentrations en LH mesurées quantitativement présentent un profil de sécrétion identique à l'évolution de l'intensité du signal coloré observé sur les bâtonnets (« sticks ») avec un maximum détecté à 11H et un minimum à 13H.

Les résultats qualitatifs obtenus avec la trousse de détection du pic de LH montrent donc une parfaite corrélation avec la quantification de la LH mesurée avec le kit LH DETECT (marque déposée). Ces résultats ont été répétés sur un effectif de 70 vaches Holstein, Montbéliardes, Charolaises, Blonde d'Aquitaine. Une corrélation de 100% a été obtenue dans toutes les races et a validé l'utilisation de la présente trousse, sur le terrain, pour une détection du pic pré ovulatoire de LH.

### b) Exemple de détection du pic préovulatoire de LH dans le sang et le mucus vaginal chez une vache Holstein.

Cet exemple a été réalisé en ferme, sur une vache Holstein représentative, ayant reçu un traitement de super ovulation pour la production d'embryons.

Avant le traitement, un implant CRESTAR SO (Centravet, France) a été posé au 8^{ème} jour du cycle sexuel et retiré au soir du 12^{ème} jour.

Le traitement de super ovulation a débuté au 10^{ème} jour du cycle et a été conduit selon les instructions du fabricant. Il a consisté classiquement en 8 injections respectivement de 1,75 ml - 1,75 ml - 1,5 ml - 1,25 ml - 1,25 ml - 1 ml - 0,75 ml - 0,75 ml de Stimufol (marque déposée, société ULg FMV PhR, Belgique) par voie intra-musculaire espacées de 12 heures (FSH1 à FSH8) et étalées sur quatre jours de J10 à J13. Une injection par voie intra-musculaire de 3 ml de Prosolvin (marque déposée, société Virbac, France) a également été réalisée à FSH5.

Des prises de sang et de mucus vaginal ont été réalisées au 13^{ème} jour à 7H-13H-19H, au 14^{ème} jour à 7H-11h-16H et au 15^{ème} jour à 7H.

La collecte du mucus vaginal a été réalisée à l'aide d'une seringue stérile de 20 ou 50 ml, reliée à un cathéter stérile (référence GAI 405, commercialisé par la société Centravet, France). Le prélèvement est ensuite récupéré dans 1 ml (millilitre) de PBS pH 7,4 (K₂HPO₄/KH₂PO₄ 0,01M - NaCl 0,15M; fournisseur VWR, ref. 26930.293, 26936.293 et 27810.295).

Les collectes de sang et de mucus vaginal ont été réalisées au même moment afin de pouvoir comparer la cinétique d'apparition du pic de LH dans les deux milieux.

Les prélèvements de sang et de mucus ont été analysés qualitativement, c'est-à-dire l'intensité du signal a été mesurée selon le procédé précité et quantitativement avec le kit de dosage LH DETECT (marque déposée).

Les résultats sont illustrés sur la Figure 20 et sur le tableau 16.

La figure 20A représente des photographies de bâtonnets (« sticks ») obtenus selon le procédé de l'invention à partir des prélèvements de sang et de mucus vaginal de la vache au treizième (J13) et au quatorzième (J14) jours du cycle.

La figure 20B représente la concentration en LH en ng/ml en fonction du temps en heures des différents prélèvements réalisés au treizième jour (J13), et au quatorzième jour (J14) et quinzième jour (J15). Tableau 16. Valeurs de la concentration en LH et de l'intensité du signal coloré des bâtonnets (« sticks ») obtenus à partir des différents prélèvements de sang et de mucus vaginal.

| prélèvements heure et date | | Concentration en LH | Quantification du signal coloré des bâtonnets (« sticks ») | |
|---|---|---|---|---|
| | | (ng/ml) | Sang | Mucus vaginal |
| 7H | J13 | 0,1 | 5,5 | 7,4 |
| 13H | J13 | 0,13 | 5,3 | 2,2 |
| 19H | J13 | 0,14 | 5,6 | 6,3 |
| 7H | J14 | 0,67 | 5,7 | 7,3 |
| 11H | J14 | 17,57 | 13,4 | 22,7 |
| 15H | J14 | 3,27 | 5,8 | 11,5 |

Au cours des prélèvements à J13, aucun signal coloré n'a été observé sur les bâtonnets (« sticks ») que ce soit dans le sang ou dans le mucus vaginal. Ces résultats corrèlent avec les valeurs très basses de concentration plasmatique de LH démontrant ainsi qu'il n'y a aucun faux positif. Au cours de J14, un signal coloré intense a été observé sur les bâtonnets (« sticks ») à 11H à la fois sur le sang et sur le mucus vaginal, correspondant au maximum du pic de sécrétion de LH (17,57 ng/ml) mesuré avec LH DETECT (marque déposée). Un signal coloré plus faible a été observé sur les bâtonnets (« sticks ») à 15H sur le sang et le mucus vaginal, correspondant à la fin du pic de sécrétion de LH (3,27 ng/ml) mesuré avec LH DETECT (marque déposée). L'ovulation a été constatée par échographie le lendemain à J15, témoignant bien d'un pic de LH fonctionnel 24 heures plus tôt, à J14.

Ces résultats démontrent très clairement que le procédé de l'invention et la trousse pour la mise en oeuvre de ce procédé permettent de détecter le pic pré ovulatoire de LH à partir d'échantillon de sang total mais également à partir d'échantillon de mucus vaginal. L'apparition du pic pré ovulatoire de LH est constatée de façon synchrone dans les deux échantillons.

Avantageusement, le procédé ainsi que la trousse de l'invention permet de détecter très facilement, en ferme, le pic de LH pré ovulatoire de LH et constitue en cela un outil d'amélioration de la pratique de l'insémination artificielle chez la vache. Cette trousse est en cela un outil de prédiction du moment de l'ovulation (intervenant, chez la vache, 24 heures après le pic de LH) permettant ainsi, en élevages, de mieux planifier l'acte d'insémination artificielle.

La trousse peut être utilisée en support des traitements de super ovulation pour optimiser la fécondation et l'obtention d'un nombre important d'embryons de bonne qualité, transférables.

### Exemple 4: mise en oeuvre du procédé de détection du pic de LH chez le porcin dans différents milieux biologiques

Le procédé de détection du pic de LH a été mise en oeuvre à partir de différents d'échantillons de l'espèce porcine: le sang, le plasma, le sérum et le mucus vaginal. Les essais ont porté sur des truies Large-White multipares élevées en bandes, dans des conditions d'élevage intensif.

Dans chaque essai, les résultats obtenus avec le procédé de l'invention ont été systématiquement corrélés avec un dosage ELISA quantitatif réalisé sur les mêmes échantillons de sang à l'aide du kit LH DETECT (marque déposée) (ReproPharm SA, France). Pour la présentation des résultats ci-dessous, la lecture visuelle des tests a été validée par une quantification densitométrique de la couleur des bâtonnets (« sticks »). Pour cela, les bâtonnets (« sticks ») ont été scannés à l'aide d'un Scanner EPSON (Perfection 1200 PHOTO) puis l'intensité de la couleur obtenue sur chacun d'eux est quantifiée par densitométrie avec le logiciel « Scion Image » (Scion Incorporation). Cette quantification est exprimée en unités de densité.

Les prises de sang ont été réalisées à l'aide de vacutainers héparinés au niveau de la veine jugulaire.

La collecte du mucus vaginal a été réalisée à l'aide d'une sonde d'insémination (référence Sonde Kobi, Cobiporc, France). Le prélèvement de mucus a ensuite été récupéré dans 1 ml (millilitre) de PBS pH 7,4 (K₂HPO₄/KH₂PO₄ 0,01M - NaCl 0,15M ; fournisseur VWR, ref. 26930.293, 26936.293 et 27810.295)

Les collectes de sang et de mucus vaginal ont été réalisées au même moment afin de pouvoir comparer la cinétique d'apparition du pic de LH dans les deux milieux biologiques.

Le protocole de conduite en bandes a été le suivant : les truies ont été sevrées 28 jours après avoir mis bas. Le sevrage indique le jour de départ d'un nouveau cycle (J0). Les prises de sang et de mucus ont eu lieu à 9h et 16H30 tous les jours de J3 à J7. Parallèlement, une détection des chaleurs a été réalisée à ces mêmes heures ainsi qu'une échographie des ovaires, pour dater l'ovulation.

Sur chaque échantillon, la détection du pic de LH a été réalisée à l'aide de la trousse de l'exemple 5 ci-dessous.

Des résultats obtenus avec une première truie (truie 1) sont représentés sur la Figure 21 et dans le tableau 17 ci-dessous.

La figure 21A représente des photographies de bâtonnets (« sticks ») obtenus selon le procédé de l'invention à partir du sang ou du mucus vaginal de la truie 1 au quatrième jour (J4) et cinquième jour (J5) après sevrage.

La figure 21B représente la concentration plasmatique en LH en ng/ml en fonction du temps en heures au quatrième jour (J4) et cinquième jour (J5) après sevrage.

**Tableau 17. Valeurs de la concentration en LH et de l'intensité du signal coloré des bâtonnets (« sticks ») obtenus à partir des différents prélèvements de sang et de mucus vaginal.**

| prélèvements heure et date | | Concentration en LH | Quantification du signal coloré des bâtonnets (« sticks ») | |
|---|---|---|---|---|
| | | (ng/ml) | Sang | Mucus vaginal |
| 9H | J4 | 9,27 | 10,52 | 20,33 |
| 16H30 | J4 | 13,45 | 12,12 | 26,92 |
| 9H | J5 | 8,79 | 7,35 | 5,89 |
| 16H30 | J5 | 5,8 | 4,29 | 4,72 |

Dans les deux milieux biologiques, les bâtonnets (« sticks ») ont montré un signal coloré à J4, indiquant un début de pic de LH à 9H et un maximum de sécrétion à 16H30 pour lequel le signal coloré a été le plus intense dans le sang comme dans le mucus vaginal. A J5, les bâtonnets (« sticks ») ont donné un léger signal coloré à 9H puis aucune couleur à 16H30 indiquant un retour à une concentration basale de LH. Cette détection qualitative est parfaitement corrélée aux valeurs des concentrations obtenues par le dosage quantitatif.

La validité des résultats obtenus avec le procédé de l'invention a été renforcée par les résultats d'échographie obtenus indiquant une ovulation à 9H J6, soit 48H après le début du pic de LH

Les résultats obtenus avec une deuxième truie (truie 2) sont représentés dans la figure 22 et le tableau 18.

La figure 22A représente des photographies de bâtonnets (« sticks ») obtenus selon le procédé de l'invention à partir du sang ou du mucus vaginal de la truie 2au troisième jour (J3) ou quatrième jour (J4) et cinquième jour (J5) après sevrage.

La figure 22B représente la concentration plasmatique en LH en ng/ml en fonction du temps en heures au troisième jour (J3), quatrième jour (J4) et cinquième jour (J5) après sevrage.

**Tableau 18. Valeurs de la concentration en LH et de l'intensité du signal coloré des bâtonnets (« sticks ») obtenus à partir des différents prélèvements de sang et de mucus vaginal.**

| prélèvements heure et date | | Concentration en LH | Quantification du signal coloré des bâtonnets (« sticks ») | |
|---|---|---|---|---|
| | | (ng/ml) | Sang | Mucus vaginal |
| 16H30 | J3 | 2,5 | 10,59 | 26,43 |
| 9H | J4 | 4,8 | 13,93 | 47,78 |
| 16H30 | J4 | 3,5 | | |
| 9H | J5 | 0,8 | 1 | 15,34 |
| 16H30 | J5 | 0,5 | 0,48 | 0,06 |

Dans cet exemple, il a été démontré dans le sang comme dans le mucus vaginal, l'apparition d'un signal coloré sur les bâtonnets (« sticks ») à 16H30 J3 qui s'amplifie à 9H au quatrième jour (J4), puis décroît à 9H au cinquième jour (J5) pour s'annuler à 16H30 au cinquième jour (J5) (figure 22A). Ces données sont parfaitement corrélées aux valeurs des concentrations plasmatiques de LH déterminées avec le dosage quantitatif (figure 22B). Le procédé de l'invention permet d'obtenir un signal coloré sur un intervalle de 36 heures particulièrement dans le mucus vaginal, ce qui corrèle très bien avec la durée moyenne d'un pic de LH chez la truie qui varie entre 36 et 48 heures.

La validité physiologique des résultats obtenus avec le procédé de l'invention est renforcée également par les résultats d'échographie indiquant une ovulation à 16H30 au cinquième jour (J5), soit 48H après le début du pic de LH.

L'ensemble de ces résultats indique que le procédé de l'invention permet la détection du pic pré ovulatoire de LH, dans les élevages, à la fois dans le sang et dans le mucus vaginal chez la truie.

En outre tel que démontré dans cet exemple, le procédé de l'invention ainsi que la trousse de l'invention permet avantageusement une détection visuelle du pic de LH.

### Exemple 5 : fabrication d'une trousse pour la mise en oeuvre du procédé de l'invention

Dans cet exemple, la trousse comprend :
- un bâtonnet (« stick ») comprenant des surfaces tests, prêt à l'emploi dans un tube hépariné,
- un tube comprenant la solution de conjugué, à savoir une solution tampon avec un anticorps anti-LH couplé à la péroxydase de raifort (anticorps conjugué), et
- un tube comprenant une solution comprenant le substrat de la péroxydase de raifort, à savoir le TMB membrane appelé substrat.

### Matériel utilisé :

- Immunostick commercialisé par la société NUNC, et
- trois cryotubes à bouchon à vis commercialisé par la société NUNC. Tous les tampons utilisés dans la fabrication du kit ont été préparés dans de l'eau milliQ.

Les différents éléments ont été préparés selon les procédés décrits ci-dessous.

### Préparation des tubes héparinés :

Préparation par mélange de 100 µl d'une solution d'héparine sodique (Héparine Choay, à 5000UI/ml marque déposée, commercialisée par Sanofi Aventis) dans 900 µl de PBS filtré sur filtre de porosité 0,22µm (fournisseur Millipore ; référence GSWP04700).

Distribution de 6UI d'héparine sodique par tube (cryotube NUNC, Dutscher, ref. 055003) soit 12µl de solution à 500UI/ml.

Séchage 3 heures dans une étuve à 37°C.

Conservation à 4°C ou à -20°C.

### Préparation des bâtonnets (« sticks »)

Recouvrement (« coating ») du bâtonnet (« stick ») avec 250 µl d'anticorps lapin anti-LH bovine purifié sur colonne de Protéine A sépharose à partir d'un immun sérum produit chez le lapin par le prestataire Eurogentec (Belgique) selon le procédé «Standard anti-protein packages 28-day Speedy in Rabbit» (marque de commerce) dénommé ci-dessous AC1 préparé à 20 µg/ml dans du tampon NaHCO₃/Na₂CO₃ 0,1M pH 9,6 contenant 0,05% en volume de ProClin300 (marque déposée) commercialisé par la société Sigma-Aldrich, ref. 48912-U.

Dans un autre mode de réalisation l'anticorps anti-LH (AC1) était un anticorps de lapin anti-LH porcine il s'agit de l'anticorps produit, par exemple par immunisation de lapins selon le procédé décrit par exemple dans la référence bibliographique « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ;266 :2291-2294 [5]. Il s'agissait du procédé comprenant par exemple trois injections de 200 µg de LH porcine purifiée commercialisée par la société Tucker Endocrine Research Institute LLC TUENRE (Atlanta, USA), qui ont été réalisées toutes les deux semaines, suivi de dix rappels avec 100µg de LH porcine purifiée effectués toutes les cinq semaines. Les saignées ont été réalisées six et neuf jours après chaque rappel et 40 ml de sang ont été récupéré. A partir des échantillons de sang les anticorps de lapin ont été purifiés par chromatographie d'affinité sur gel de Protéine A sépharose selon le procédé décrit dans « Techniques Immuno-enzymatiques » Thérèse Ternynck et Stratis Avrameas, éditions INSERM, 1987 [1] Le couplage de l'anticorps a été réalisé selon le procédé décrit dans « Techniques Immuno-enzymatiques » Thérèse Ternynck et Stratis Avrameas, éditions INSERM, 1987 [1] Incubation une heure à 37°C, puis 18 heures à 4°C.
Essorage du bâtonnet (« stick ») par secousses.
Recouvrement (« surcoating ») du bâtonnet (« stick ») dans 900 µl de PBS additionné de Sérum de veau foetal (SVF) 50% (fournisseur LONZA, référence 14-801F) pendant 1 heure à 37°C.
Essorage du bâtonnet (« stick ») par secousses.
Séchage du bâtonnet (« stick ») à la verticale à l'étuve 37°C pendant 3 heures.
Conservation du bâtonnet (« stick ») à 4°C ou à -20°C dans un tube hépariné séché.

### Préparation du réactif comprenant la solution de conjugué (tube n°2)

Préparation de l'anticorps cheval anti-LH ovine couplé à la péroxydase de raifort (HRP), dénommé ci-dessous AC2 HRP à 10µg/ml dans du PBS SVF 50% en volume contenant 0,05% en volume de ProClin300 (marque déposée). Incubation 50 minutes à 37°C et distribution de 300 µl par cryotube. Conservation à 4°C ou à -20°C.

Cet anticorps a été produit à partir d'un anticorps purifié sur colonne de Protéine G sépharose à partir d'un sérum de cheval immunisé contre de la LH ovine purifiée. Il s'agissait en particulier de l'anticorps anti-LH ovine fait chez le cheval décrit dans le brevet français FR N° FR90 06863, N° de brevet FR 2 662 804. Cet anticorps purifié a ensuite été couplé selon le protocole décrit dans Techniques Immuno-enzymatiques de Thérèse Ternynck et Stratis Avrameas, éditions INSERM, 1987 [1]. Dans un autre mode de réalisation il s'agit de l'anticorps produit, par exemple par immunisation de lapins selon le procédé décrit dans la référence bibliographique « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ;266 :2291-2294 [5]. Il s'agissait du procédé comprenant par exemple trois injections de 200 µg de LH porcine purifiée provenant de la société Tucker Endocrine Research Institute LLCTUENRE (Atlanta, USA), qui ont été réalisées toutes les deux semaines, suivi de dix rappels avec 100µg de LH purifiée effectués toutes les cinq semaines. Les saignées ont été réalisées six et neuf jours après chaque rappel et 40ml de sang ont été récupéré. A partir des échantillons de sang les anticorps de lapin ont été purifiés par chromatographie d'affinité sur gel de Protéine A sépharose selon le procédé décrit dans « Techniques Immuno-enzymatiques » Thérèse Ternynck et Stratis Avrameas, éditions INSERM, 1987 [1] Le couplage de l'anticorps a été réalisé selon le procédé décrit dans « Techniques Immuno-enzymatiques » Thérèse Ternynck et Stratis Avrameas, éditions INSERM, 1987 [1]. La LH porcine était commercialisée par Tucker Endocrine Research Institute LLCTUENRE (Atanta, USA). La péroxydase utilisée est commercialisée par Sigma sous la référence P6782.

### Préparation des tubes de révélateur (tube n°3)

Dilution de TMB Membrane commercialisé par KPL sous la référence 50-77-18 à 50% en volume dans du tampon citrate (NaCH₃CO₂) 0,03M ajusté à pH5 avec de l'acide acétique (CH₃COOH). Distribution de 300µl du mélange par cryotube.
Conservation à 4°C ou à -20°C à l'abri de la lumière.

### Listes des références

1. « Techniques Immuno-enzymatiques » Thérèse Ternynck et Stratis Avrameas, éditions INSERM, 1987;
2. Immunobiologie Charles A. Janeway, Paul Travers, Pierre L. Masson - 2003 - Medical ; Janeway's Immunobiology, Kenneth Murphy, Paul Travers, Mark Walport, 2011, éditions GS)
3. « Enzyme-linked immunosorbent assay (ELISA). Quantitative assay of immunoglobulin G », Engvall, E. et Perlman, P., Immunochemistry, 1971 Sep;8(9):871-4 PMID: 5135623
4. « Enzyme-Linked Immunosorbent Assay » Goldsby, R.A., Kindt, T.J., Osborne, B.A. et Kuby, J., in Immunology, 5ème édition (2003), pp. 148-150., W. H. Freeman, New York.
5. « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ;266 :2291-2294
6. La production d'embryons chez les bovins : quelles voies de recherche pour augmenter l'efficacité des traitements de super ovulation, Saumande J. INRA Productions Animales, 1995 ; 8(4), 275-283.
7. "Use of Norgestomet implant as an aid when superovulatig low fertility dairy cattle", Ellington JE, Elefson EE, McCall RM. Theriogenology, 1987; 27, 227.

## Revendications

1. Procédé de détection du pic préovulatoire de LH dans un échantillon biologique provenant de mammifères et comprenant les étapes suivantes :
a. fixer d'un anticorps anti-LH sur une surface test ;
b. mettre en contact la surface test sur laquelle est fixé ledit anticorps anti-LH avec une solution tampon comprenant de 5 à 50% en volume de sérum de veau foetal ;
c. mettre en contact ladite surface obtenue à l'étape (b) avec un échantillon biologique ;
d. après l'étape (c) rincer la surface test dans une solution de lavage ;
e. mettre en contact la surface test rincée à l'étape (d) avec une solution tampon de conjugué comprenant un anticorps anti-LH couplé à une enzyme et de 5 à 50% en volume de sérum de veau foetal ;
f. après l'étape (e) rincer la surface test dans une solution de lavage ; et
g. après l'étape (f) mettre en contact la surface test avec une solution comprenant un substrat de ladite enzyme, ledit substrat étant choisi dans le groupe comprenant le 3,3'3,3',5,5'-tétramethylbenzidine (TMB membrane), le tétrahydrochlorure de 4-Chloro-1-naphto / 3,3' - diaminobenzidine (CN/DAB), le 3-amino-9-éthylcarbazole (AEC), 3,3-Diméthoxybenzidine-o-dianisidine (ODN), le 5-bromo-4-chloro-3'-indolylohosphate pToluidine (BCIP), un mélange chlorure de nitro-bleu tétrazolium (NBT) et de 5-bromo-4-chloro-3'-indolylohosphate pToluidine (BCIP), le mélange Naphtol As-Mx phosphate et sel de 4-Chloro-2-méthylbenzènediazonium (Fast red TR salt), le mélange Naphtol As-Mx phosphate et le sel diazoté de chlorure de 4'-amino-2',5'-diéthoxybenzanilide zinc (Fast blue BB salt), le 5-Iodo-3-Indolyl-β-D-galactopyranoside (Purple-Gal), le 5-Bromo-6-chloro-3-indolyl-β-D-galactopyranoside (Red-Gal), le 6-chloro-3-indolyl-β-D-galactopyranoside (Rose-Gal), le 5-bromo-3-indolyl-β-D-galactopyranoside (Blue-Gal), le N-méthylindolyl-β-D-galactopyranoside (Green-Gal).

2. Procédé selon la revendication 1, dans lequel l'échantillon biologique est choisi dans le groupe comprenant le sang, le plasma, le sérum, le mucus vaginal, la salive, l'urine, le lait.

3. Procédé selon l'une quelconque des revendications précédentes, dans lequel la solution de lavage utilisée aux étapes (d) ou (f) est le même ou pas.

4. Procédé selon l'une quelconque des revendications précédentes, dans lequel la solution de lavage utilisée aux étapes (d) ou (f) est choisie parmi un tampon phosphate salin ou de l'eau.

5. Procédé selon l'une quelconque des revendications précédentes, dans lequel le mammifère est un humain ou un animal.

6. Procédé selon l'une quelconque des revendications précédentes dans lequel l'animal est choisi dans le groupe comprenant les bovins, porcins, ovins, caprins, canins, équins.

7. Procédé selon l'une quelconque des revendications 1 à 5, dans lequel le mammifère est un humain.

8. Procédé selon l'une quelconque des revendications précédentes, dans lequel l'anticorps anti-LH fixé sur la surface test à l'étape (a) est un anticorps polyclonal ou monoclonal choisi dans le groupe comprenant l'anticorps anti-LH bovine, l'anticorps anti-LH porcine, l'anticorps anti-LH ovine, l'anticorps anti-LH canine, l'anticorps anti-LH féline, l'anticorps anti-LH équine, l'anticorps anti-LH cameline, l'anticorps anti-LH humaine.

9. Procédé selon l'une quelconque des revendications précédentes, dans lequel l'anticorps anti-LH couplé à l'enzyme est un anticorps polyclonal ou monoclonal choisi dans le groupe comprenant l'anticorps anti-LH bovine, l'anticorps anti-LH porcine, l'anticorps anti-LH ovine, l'anticorps anti-LH canine, l'anticorps anti-LH féline, l'anticorps anti-LH équine, l'anticorps anti-LH cameline, l'anticorps anti-LH humaine.

10. Procédé selon l'une quelconque des revendications précédentes, dans lequel l'anticorps anti-LH est couplé à une enzyme choisie dans le groupe comprenant l'enzyme peroxydase, la beta-galactosidase, la glucose oxydase et la phosphatase alcaline.

11. Procédé selon la revendication 10, dans lequel l'enzyme est la péroxydase.

12. Procédé selon la revendication 11, dans lequel la péroxydase est choisie dans le groupe comprenant les péroxydases à hème ou les péroxydases sans hème.

13. Procédé selon l'une quelconque des revendications précédentes, dans lequel l'étape (c) et (e) de mise en contact est réalisée pendant au moins 5 minutes.

14. Procédé selon l'une quelconque des revendications précédentes, dans lequel l'étape (g) de mise en contact est réalisée pendant au moins 5 minutes.

15. Procédé selon l'une quelconque des revendications 11 à 13, dans lequel à l'étape (f) le substrat est le TMB membrane.

16. Procédé selon la revendication 15, dans lequel le TMB membrane est dilué dans un tampon citrate avec un facteur de dilution de 1/2 à 1/20

17. Procédé selon l'une quelconque des revendications précédentes dans lequel la surface test est en plastique.

18. Procédé selon l'une quelconque des revendications précédentes, dans lequel l'échantillon biologique est du sang et l'étape (b) de mise en contact est réalisée dans un tube hépariné.

19. Trousse pour la mise en oeuvre du procédé selon l'une quelconque des revendications 1 à 18, comprenant un support présentant une surface test sur laquelle des anticorps anti-LH sont fixés, un tampon PBS, une solution tampon de conjugué comprenant du sérum de veau foetal et un anticorps anti-LH couplé à une enzyme, et, une solution tampon de révélation comprenant un substrat de ladite enzyme, ledit substrat étant choisi dans le groupe comprenant le 3,3'3,3',5,5'-tétramethylbenzidine (TMB membrane), le tétrahydrochlorure de 4-Chloro-1-naphto / 3,3' - diaminobenzidine (CN/DAB), le 3-amino-9-éthylcarbazole (AEC), 3,3-Diméthoxybenzidine-o-dianisidine (ODN), le 5-bromo-4-chloro-3'-indolylohosphate pToluidine (BCIP), un mélange chlorure de nitro-bleu tétrazolium (NBT) et de 5-bromo-4-chloro-3'-indolylohosphate pToluidine (BCIP), le mélange Naphtol As-Mx phosphate et sel de 4-Chloro-2-méthylbenzènediazonium (Fast red TR salt), le mélange Naphtol As-Mx phosphate et le sel diazoté de chlorure de 4'-amino-2',5'-diéthoxybenzanilide zinc (Fast blue BB salt), le 5-Iodo-3-Indolyl-β-D-galactopyranoside (Purple-Gal), le 5-Bromo-6-chloro-3-indolyl-β-D-galactopyranoside (Red-Gal), le 6-chloro-3-indolyl-β-D-galactopyranoside (Rose-Gal), le 5-bromo-3-indolyl-β-D-galactopyranoside (Blue-Gal), le N-méthylindolyl-β-D-galactopyranoside (Green-Gal).

20. Trousse selon la revendication 19, dans lequel ledit support est un bâtonnet (« stick ») avec plusieurs surfaces tests.

21. Trousse selon la revendication 19 ou 20, dans laquelle l'anticorps anti-LH est couplé à la péroxydase et ladite solution tampon de révélation comprend du TMB membrane.

## Patentansprüche

1. Verfahren zur Bestimmung des präovulatorischen LH-Anstiegs in einer biologischen Probe von Säugetieren die folgenden Schritte umfassend:
a. Fixieren eines Anti-LH-Antikörpers auf einer Testoberfläche;
b. In-Kontakt-bringen der Testoberfläche, auf welcher der Anti-LH-Antikörper fixiert ist, mit einer Pufferlösung, die von 5 bis 50 Vol% fötales Kälberserum enthält;
c. In-Kontakt-bringen der in Schritt (b) erhaltenen Oberfläche mit einer biologischen Probe;
d. Nach dem Schritt (c) Spülen der Testoberfläche in einer Waschlösung;
e. In-Kontakt-bringen der in Schritt (d) gespülten Testoberfläche mit einer Konjugat-Pufferlösung, die einen Anti-LH-Antikörper umfasst, der an ein Enzym gekoppelt ist, und von 5 bis 50 Vol-% fötales Kälberserum umfasst;
f. Nach dem Schritt (e) Spülen der Testoberfläche in einer Waschlösung; und
g. Nach dem Schritt (f) In-Kontakt-bringen der Testoberfläche mit einer Lösung, die ein Substrat des Enzyms umfasst, wobei das Substrat aus der Gruppe ausgewählt ist, die folgende umfasst: 3,3'3,3',5,5'-Tetramethylbenzidin (TMB-Membran), 4-Chlor-1-naphtho/3,3,-Diaminobenzidin- Tetrahydrochlorid (CN/DAB), 3-Amino-9-ethylcarbazol (AEC), 3,3-Dimethoxybenzidin-o-Dianisidin (ODN), 5-Brom-4-chlor-3'indolylphosphat pToluidin (BCIP), ein Gemisch von Nitroblautetrazolium-Chlorid (NBT) und 5-Brom-4-chlor-3'indolylphosphat pToluidine (BCIP), das Gemisch von Naphthol-AS-MX-phosphat und Salz von 4-Chlor-2-methylbenzendiazonium (Fast red TR Salz), das Gemisch von Naphthol AS-MX-Phosphat und diazotiertem Salz von 4'-Amino-2',5'-diethoxybenzanilid-Zinkchlorid (Fast Blue BB Salz), 5-Iodo-3-indolyl-β-D-galactopyranosid (Purple-Gal), 5-Brom-6-chlor-3-indolyl-β-D-galactopyranosid (Red-Gal), 6-Chlor-3-indolyl-β-D-galactopyranosid (Rose-Gal), 5-Bromo-3-indolyl-β-D-galactopyranosid (Blue-Gal), N-Methylindolyl-β-D-galactopyranosid (Green-Gal).

2. Verfahren nach Anspruch 1, wobei die biologische Probe aus der Gruppe ausgewählt ist, die Blut, Plasma, Serum, Vaginalschleim, Speichel, Urin, Milch umfasst.

3. Verfahren nach einem der vorhergehenden Ansprüche, wobei die Waschlösung, die in den Schritten (d) oder (f) verwendet wird, die gleiche ist oder nicht.

4. Verfahren nach einem der vorhergehenden Ansprüche, bei dem die Waschlösung, die in den Schritten (d) oder (f) verwendet wird, aus phosphatgepufferter Salzlösung oder Wasser ausgewählt ist.

5. Verfahren nach einem der vorhergehenden Ansprüche, wobei das Säugetier ein Mensch oder ein Tier ist.

6. Verfahren nach einem der vorhergehenden Ansprüche, wobei das Tier aus der Gruppe ausgewählt ist, die Rinder, Schweine, Schafe, Ziegen, Hunde, Pferde umfasst.

7. Verfahren nach einem der Ansprüche 1 bis 5, wobei der Säuger ein Mensch ist.

8. Verfahren nach einem der vorhergehenden Ansprüchen, wobei der an der Testoberfläche in Schritt (a) fixierte Anti-LH-Antikörper ein polyklonaler oder monoklonaler Antikörper ist, der aus der Gruppe ausgewählt ist, die folgende umfasst: Anti-LH-Antikörper des Rinds, Anti-LH-Antikörper des Schweins, Anti-LH-Antikörper des Schafs, Anti-LH-Antikörper des Hunds, Anti-LH-Antikörper der Katze, Anti-LH-Antikörper des Pferds, Anti-LH-Antikörper des Kamels, humane Anti-LH-Antikörper.

9. Verfahren nach einem der vorhergehenden Ansprüchen, wobei der an ein Enzym gekoppelte Anti-LH-Antikörper ein polyklonaler oder monoklonaler Antikörper ist, der aus der Gruppe ausgewählt ist, die folgende umfasst: Anti-LH-Antikörper des Rinds, Anti-LH-Antikörper des Schweins, Anti-LH-Antikörper des Schafs, Anti-LH-Antikörper des Hunds, Anti-LH-Antikörper der Katze, Anti-LH-Antikörper des Pferds, Anti-LH-Antikörper des Kamels, humane Anti-LH-Antikörper.

10. Verfahren nach einem der vorhergehenden Ansprüche, wobei der Anti-LH-Antikörper an ein Enzym gekoppelt ist, ausgewählt aus der Gruppe, die Peroxidase-Enzym, beta-Galactosidase, Glucoseoxidase und alkalische Phosphatase umfasst.

11. Verfahren nach Anspruch 10, wobei das Enzym Peroxidase ist.

12. Verfahren nach Anspruch 11, wobei die Peroxidase aus der Gruppe ausgewählt ist, die Peroxidasen mit oder ohne Häme umfasst.

13. Verfahren nach einem der vorhergehenden Ansprüche, wobei der Schritt (c) und (e) des In-Kontakt-Bringens während mindestens 5 Minuten durchgeführt wird.

14. Verfahren nach einem der vorhergehenden Ansprüche, wobei der Schritt (g) des In-Kontakt-Bringens während mindestens 5 Minuten durchgeführt wird.

15. Verfahren nach einem der Ansprüche 11 bis 13, wobei in Schritt (f) das Substrat TMB-Membran ist.

16. Verfahren nach Anspruch 15, wobei die TMB-Membran in einem Citratpuffer mit einem Verdünnungsfaktor von 1/2 bis 1/20 verdünnt ist.

17. Verfahren nach einem der vorhergehenden Ansprüche, wobei die Testoberfläche aus Kunststoff ist.

18. Verfahren nach einem der vorhergehenden Ansprüche, wobei die biologische Probe Blut ist und der Schritt (b) des In-Kontakt-Bringens in einem heparinisierten Röhrchen durchgeführt wird.

19. Kit zur Durchführung des Verfahrens nach einem der Ansprüche 1 bis 18, umfassend einen Träger mit einer Testoberfläche, auf der Anti-LH-Antikörper gebunden sind, einen PBS-Puffer, eine Konjugat-Pufferlösung, umfassend fötales Kälberserum und einen an ein Enzym gekoppelten Anti-LH-Antikörper, und eine Nachweis-Pufferlösung, die ein Substrat des Enzyms umfasst, wobei das Substrat aus der Gruppe ausgewählt ist, die folgende umfasst: 3,3'3,3',5,5'-Tetramethylbenzidin (TMB-Membran), 4-Chlor-1-naphtho/3,3,-Diaminobenzidin-Tetrahydrochlorid (CN/DAB), 3-Amino-9-ethylcarbazol (AEC), 3,3-Dimethoxybenzidin-o-Dianisidin (ODN), 5-Brom-4-chlor-3'indolylphosphat pToluidin (BCIP), ein Gemisch von Nitroblautetrazolium-Chlorid (NBT) und 5-Brom-4-chlor-3'indolylphosphat pToluidine (BCIP), das Gemisch von Naphthol-AS-MX-phosphat und Salz von 4-Chlor-2-methylbenzendiazonium (Fast red TR Salz), das Gemisch von Naphthol AS-MX-Phosphat und diazotiertem Salz von 4'-Amino-2',5'-diethoxybenzanilid-Zinkchlorid (Fast Blue BB Salz), 5-Iodo-3-indolyl-β-D-galactopyranosid (Purple-Gal), 5-Brom-6-chlor-3-indolyl-β-D-galactopyranosid (Red-Gal), 6-Chlor-3-indolyl-β-D-galactopyranosid (Rose-Gal), 5-Bromo-3-indolyl-β-D-galactopyranosid (Blue-Gal), N-Methylindolyl-β-D-galactopyranosid (Green-Gal).

20. Kit nach Anspruch 19, wobei der Träger ein Stäbchen ("stick") mit mehreren Testoberflächen ist.

21. Kit nach Anspruch 19 oder 20, wobei der Anti-LH-Antikörper mit Peroxidase gekoppelt ist, und die Nachweis-Pufferlösung TMB-Membran umfasst.

## Claims

1. A method of detecting the preovulatory LH peak in a biological sample obtained from mammals and comprising the following steps:
a. fixing an anti-LH antibody on a test surface;
b. contacting the test surface on which said anti-LH antibody is fixed with a buffer solution comprising 5 to 50 vol % of fetal calf serum;
c. contacting said surface obtained in step (b) with a biological sample
d. after step (c), rinsing the test surface in a washing solution;
e. contacting the test surface rinsed in step (d) with a buffer solution of conjugate comprising an enzyme-coupled anti-LH antibody and from 5 to 50 vol % of fetal calf serum;
f. after step (e), rinsing the test surface in a washing solution; and
g. after step (f), contacting the test surface with a solution comprising a substrate of said enzyme, said substrate being selected from the group comprising 3,3',3,3',5,5'-tetramethylbenzidine (Membrane TMB), 4-chloro-1-naphtho/3,3'-diaminobenzidine tetrahydrochloride (CN/DAB), 3-amino-9-ethylcarbazole (AEC), 3,3-dimethoxybenzidine-o-dianisidine (ODN), 5-bromo-4-chloro-3'-indolyl phosphate p-toluidine salt (BCIP), a mixture of nitro-blue tetrazolium chloride (NBT) and of 5-bromo-4-chloro-3'-indolyl phosphate p-toluidine salt (BCIP), the mixture Naphthol As-Mx phosphate and 4-chloro-2-methylbenzene diazonium salt (Fast red TR salt), the mixture Naphthol As-Mx phosphate and the diazotized salt of 4'-amino-2',5'-diethoxybenzanilide zinc chloride (Fast blue BB salt), 5-iodo-3-indolyl-β-D-galactopyranoside (Purple-Gal), 5-bromo-6-chloro-3-indolyl-β-D-galactopyranoside (Red-Gal), 6-chloro-3-indolyl-β-D-galactopyranoside (Rose-Gal), 5-bromo-3-indolyl-β-D-galactopyranoside (Blue-Gal), N-methylindolyl-β-D-galactopyranoside (Green-Gal).

2. The method according to claim 1, wherein the biological sample is selected from the group comprising blood, plasma, serum, vaginal mucus, saliva, urine, milk.

3. The method according to any preceding claims, wherein the washing solution used in steps (d) or (f) is or is not the same.

4. The method according to any preceding claims, wherein the washing solution used in steps (d) or (f) is selected from phosphate-buffered saline or water.

5. The method according to any preceding claims, wherein the mammal is a human or an animal.

6. The method according to any preceding claims, wherein the animal is selected from the group comprising bovines, pigs, sheep, goats, canines, and equines.

7. The method according to any of claims 1 to 5, wherein the mammal is a human.

8. The method according to any preceding claims, wherein the anti-LH antibody fixed on the test surface in step (a) is a polyclonal or monoclonal antibody selected from the group comprising anti-bovine LH antibody, anti-porcine LH antibody, anti-ovine LH antibody, anti-canine LH antibody, anti-feline LH antibody, anti-equine LH antibody, anti-camelid LH antibody, anti-human LH antibody.

9. The method according to any preceding claims, wherein the enzyme-coupled anti-LH antibody is a polyclonal or monoclonal antibody selected from the group comprising anti-bovine LH antibody, anti-porcine LH antibody, anti-ovine LH antibody, anti-canine LH antibody, anti-feline LH antibody, anti-equine LH antibody, anti-camelid LH antibody, anti-human LH antibody.

10. The method as claimed according to any preceding claims, wherein the anti-LH antibody is coupled to an enzyme selected from the group comprising the enzyme peroxidase, beta-galactosidase, glucose oxidase and alkaline phosphatase.

11. The method according to claim 10, wherein the enzyme is peroxidase.

12. The method according to claim 11, wherein the peroxidase is selected from the group comprising the peroxidases with heme or the peroxidases without heme.

13. The method according to any preceding claims, wherein step (c) and (e) of contacting is carried out for at least 5 minutes.

14. The method according to claim 1, wherein step (g) of contacting is carried out for at least 5 minutes.

15. The method according to any of claims 11 to 13, wherein in step (f) the substrate is Membrane TMB.

16. The method according to claim 15, wherein the Membrane TMB is diluted in a citrate buffer with a dilution factor from 1/2 to 1/20.

17. The method according to any preceding claims, wherein the test surface is of plastic.

18. The method according to any preceding claims, wherein the biological sample is blood and step (b) of contacting is carried out in a heparinized tube.

19. A kit for applying the method according to any claims 1 to 18, comprising a support having a test surface on which anti-LH antibodies are fixed, a PBS buffer, a buffer solution of conjugate comprising fetal calf serum and an enzyme-coupled anti-LH antibody, and a development buffer solution comprising a substrate of said enzyme, said substrate being selected from the group comprising 3,3',3,3',5,5'-tetramethylbenzidine (Membrane TMB), 4-chloro-1-naphtho/3,3'-diaminobenzidine tetrahydrochloride (CN/DAB), 3-amino-9-ethylcarbazole (AEC), 3,3-dimethoxybenzidine-o-dianisidine (ODN), 5-bromo-4-chloro-3'-indolyl phosphate p-toluidine salt (BCIP), a mixture of nitro-blue tetrazolium chloride (NBT) and of 5-bromo-4-chloro-3'-indolyl phosphate p-toluidine salt (BCIP), the mixture Naphthol As-Mx phosphate and 4-chloro-2-methylbenzene diazonium salt (Fast red TR salt), the mixture Naphthol As-Mx phosphate and the diazotized salt of 4'-amino-2',5'-diethoxybenzanilide zinc chloride (Fast blue BB salt), 5-iodo-3-indolyl-β-D-galactopyranoside (Purple-Gal), 5-bromo-6-chloro-3-indolyl-β-D-galactopyranoside (Red-Gal), 6-chloro-3-indolyl-β-D-galactopyranoside (Rose-Gal), 5-bromo-3-indolyl-β-D-galactopyranoside (Blue-Gal), N-methylindolyl-β-D-galactopyranoside (Green-Gal).

20. The kit according to claim 19, wherein said support is a stick with several test surfaces.

21. The kit according to claim 19 or 20, wherein the anti-LH antibody is peroxidase-coupled and said development buffer solution comprises Membrane TMB.
